# EUROPEAN PATENT SPECIFICATION

(11) **EP 0 440 324 B1**
(45) Date of publication and mention of the grant of the patent: **12.04.2000**
(21) Application number: 91300005.5
(22) Date of filing: 02.01.1991
(51) Int. Cl.: A61K 31/255, A61K 31/165, A61K 31/12, A61K 31/345, A61K 31/38, A61K 31/44, A61K 31/47, A61K 31/19, A61K 31/275, C07C 309/73, C07C 309/66, C07C 49/567, C07C 65/38, C07C 235/84, C07C 205/45, C07C 49/235, C07C 49/255, C07D 307/71, C07D 333/44, C07D 213/50, C07D 215/14, C07C 65/40, C07C 49/577, C07C 255/56, C07D 333/28

(54) **Substituted beta-diketones and their use in the treatment of inflammatory bowel disease**
Substituierte beta-Diketone und deren Verwendung zur Behandlung von entzündlichen Eingeweidenerkrankungen
Béta -dicetones disubstitués et leur utilisation dans le traitement des affections intestinales inflammatoires

(30) Priority: 02.02.1990 GB 9002337
(43) Date of publication of application: 07.08.1991
(73) Proprietor: ORION-YHTYMÄ OY, 02101 Espoo (FI)
(72) Inventor: Backstrom, Reijo Johannes, FI-00750 Helsinki (FI); Honkanen, Erkki Juhani, FI-01400 Vantaa (FI); Pystynen, Jarmo Johan, FI-02260 Espoo (FI); Luiro, Anne Maria, FI-00800 Helsinki (FI); Aho, Paivi Annikki, FI-00100 Helsinki (FI); Linden, Inge-Britt Yvonne, FI-00210 Helsinki (FI); Nissinen, Erkki Aarne Olavi, FI-02170 Espoo (FI); Pohto, Pentti, FI-00530 Helsinki (FI)
(74) Representative: Sexton, Jane Helen

(56) References cited:
- EP-A- 0 008 458
- EP-A- 0 052 300
- EP-A- 0 323 162
- DE-A- 2 210 633
- DE-A- 3 303 066
- FR-A- 2 607 493
- US-A- 3 280 069
- BULL. CHEM. SOC. JAPAN vol. 48, no. 3, 1975, pages 882 - 884 T. KAWATO ET AL
- BULL. CHEM. SOC. JAPAN vol. 49, no. 5, 1976, pages 1369 - 1374 M. IWATA ET AL
- BULL. CHIM. SOC. FRANCE no. 7-8, 1974, pages 1683 - 1690 J.-P. VECCHIONACCI ET
- EUR. J. MED. CHEM. vol. 23, no. 2, 1988, pages 111 - 117 J.R. DIMMOCK ET AL
- CHEM. PHARM. BULL. vol. 34, no. 4, 1986, pages 1619 - 1627 I. KATSUMI ET AL

## Description

The present invention relates to substituted β-diketones and their physiologically acceptable salts and esters, which are useful in the treatment of inflammatory bowel diseases.

DE-A-2,210,663 discloses 3'-nitro-4'-chlorobenzylidene-acetyl-acetone as an α,β-unsaturated dioxo compound which exhibits a cardiac relaxing and hypertensive effect.

DE-A-3,303,066 discloses substituted 1,4-dihydropyridines for the prevention of cardiovascular disorders and for treating troubles of the cerebral and peripheral blood circulation.

Bull. Chem. Soc. Japan, 48(3), 1975, pp. 882-884, T. Kawato *et al*., discloses the property of chelation of the compounds 3-(p-hydroxybenzylidene)-2,4-pentadione and 3-(m-hydroxybenzylidene)-2,4-pentadione.

Bull. Chem. Soc. Japan, 49(59, 1976, pp. 1368-1374, M. Iwata *et al*., refers to the condensation products of aromatic aldehydes with ketone promoted by Cu-II, including 3-(2-methoxybenzylidene)-2,4-pentanedione.

EP-A-0,008,458 discloses insecticidal triazole compounds useful in the protection of plants and crops.

US-A-3,280,069 is concerned with the stability of plastic against UV-light and mentions the compound 3-(o-hydroxybenzylidene)-2,4-pentanedione.

Bull. Chim. Soc. France, No. 7-8, 1974, pp. 1683-1690, J.-P. Vecchionacci *et al*., discloses the compound benzylidene-3-hexane-2,4-dione (cf. page 1688, table IV).

Eur. J. Med. Chem. 22(2), 1988, pp. 111-117, J.R. Dimmock *et al*., discloses in its experiments protocol the compounds 4-(phenylmethylene)-3,5-heptanedione and 4-(3,4-dichlorophenylmethylene)-3,5-heptandione.

EP-A-0357403 discloses β-diketones which possess cytoprotective efficacy in the stomach and in the duodenum and are useful in the treatment or prophylaxis of gastric and duodenal ulcers.

It has now been found that some substituted β-diketones are especially useful in the treatment of inflammatory bowel diseases (IBD).

The term IBD encompasses chronic inflammatory conditions of the gastrointestinal tract such as Crohn's disease and ulcerative colitis. The exact cause of the disease is still unknown. Infectious agents, immunological abnormalities, permeability disorders and leukotrienes are some of the factors suggested to play a role in the pathogenesis of IBD. The present medical therapy (sulfasalazine, 5-ASA, corticosteroids) is focused only on the diminishing of the inflammatory response, and there is still a great requirement for a more specific and effective drug for the treatment of IBD (Sutherland, CMAJ 137:799-802, 1987).

The present invention provides use of a compound of general formula I 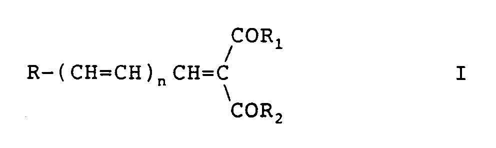 wherein n=0 or 1, R₁ and R₂ are independently methyl, ethyl or cyclopropyl and R is an optionally substituted phenyl or heteroaryl group; 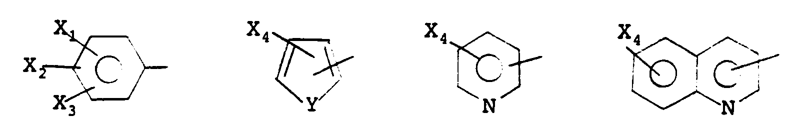 wherein X₁ is hydrogen, hydroxy or alkoxy which is optionally substituted by aryl; X₂ and X₃ are independently hydrogen, nitro, cyano, halo, trifluoromethyl, formyl, carboxy, acetamido or;

R₃S(O)ₘ

wherein m=0, 1 or 2 and R₃ is alkyl, aryl or aralkyl; or carbamoyl which is optionally substituted by alkyl, aryl or aralkyl; Y is oxygen or sulfur and X₄ is hydrogen, nitro or halo; or a pharmaceutically acceptable salt or ester thereof in the manufacture of a medicament for use in the treatment of inflammatory bowel disease.

The present invention also provides a compound according to the general formula II 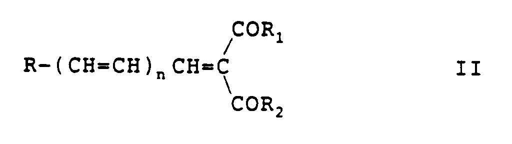 wherein n=0 or 1, R₁ is methyl, ethyl or cyclopropyl and R₂ is ethyl or cyclopropyl and R is 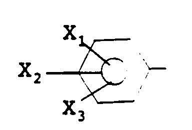 wherein X₁ is hydrogen, hydroxy or alkoxy which is optionally substituted by aryl; X₂ and X₃ are independently hydrogen, nitro, cyano, halo, trifluoromethyl, formyl, carboxy, acetamido or;

R₃S(O)ₘ

wherein m=0, 1 or 2 and R₃ is alkyl, aryl or aralkyl; or carbamoyl which is optionally substituted by alkyl, aryl or aralkyl or R₁ and R₂ are methyl and R is 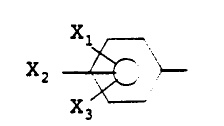 where X₁, X₂ and X₃ are as defined above provided that one of X₂ and X₃ is carbamoyl which is optionally substituted by alkyl, aryl or aralkyl, or a pharmaceutically acceptable salt or ester thereof.

The invention also provides a new compound according to the general formula III 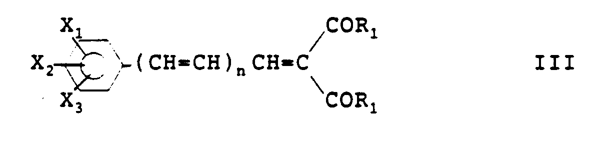 wherein n is 0 or 1, R₁ is methyl and X₁ is as defined above and X₂ is

R₃S(O)ₘ

wherein m is 1 or 2 and R₃ is alkyl, aryl or aralkyl and X₃ is hydrogen, nitro, cyano, halo, trifluoromethyl, formyl, carboxy or a group as defined for X₂; or X₂ and X₃ are both nitro or cyano; or X₂ is nitro and X₃ is formyl or halo or, when X₁ is hydroxy, X₂ may be carboxy, or X₂ and X₃ may both be hydrogen when n=0 and X₁ is alkoxy substituted by aryl, or when n=1 and X₁ is hydroxy or alkoxy optionally substituted by aryl;
or a pharmaceutically acceptable salt or ester thereof but excluding 4-(phenylmethylene)-3,5-heptanedione, 4-(3,4-dichlorophenylmethylene)-3,5-heptanedione 3-[(2-chloro-5-nitrophenyl)methylene]-2,4-pentanedione, and 3-(phenylmethylene)-2,4-hexanedione.

The term "alkyl" as employed herein refers to an alkyl group having preferably 1 to 2 carbon atoms.

The term "alkoxy" as employed herein refers to an alkyl residue as defined above linked to an oxygen atom.

The term "aralkyl" as employed herein refers to an alkyl group as defined above having aryl, preferably phenyl as the substituent.

The term "aryl" as employed herein refers to a carbocyclic aromatic group containing from 6 to 10 carbon atoms in the ring portion. The group may be a single ring or fused rings. Specific examples are phenyl and naphthyl.

The term "heteroaryl" as employed herein refers to a monocyclic or bicyclic aromatic group of carbon atoms containing one or more heteroatoms such as nitrogen, oxygen or sulfur.

The preparation of compounds according to the formulae I and II may be carried out in a similar way as described in EP-A-0357403. For example the compounds of formula I may be prepared by reacting a compound of the formula IV

R₁-CO-CH₂-CO-R₂ IV

in which R₁ and R₂ are as defined above with a compound of formula V

R-(CH=CH)ₙ-Z V

in which n and R are as defined above and Z is CHO or -CH₂-Q, wherein Q is halogen or some other activated group, in the presence of acidic or basic catalyst to produce a compound of formula I or a compound of formula VI 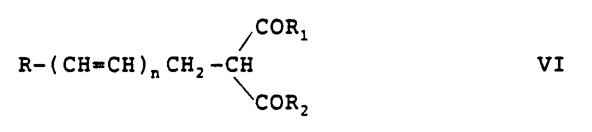 in which n, R₁, R₂ and R are as defined above, which compound is halogenated to give a compound of the formula VII 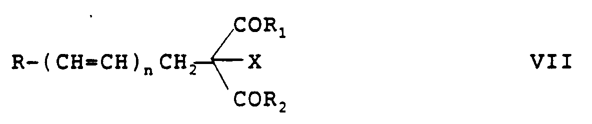 in which n, R₁, R₂ and R are as defined above and X is halogen and dehydrohalogenating compound VII to produce a compound of the formula I.

The basic catalyst may be for example an organic or inorganic base. The acidic catalyst may be for example a mineral or sulfonic acid. The activated group Q may be a halogen or an alkyl or aryl sulfonate.

When halogenating a compound of formula VI elementary halogen, preferably chlorine or bromine may be used, or another known halogenating agent such as sulfuryl chloride may be used.

Salts of these compounds may be prepared by known methods. Most commonly used physiologically acceptable salts are sodium, potassium, ammonium, calcium and magnesium salts and hydrochlorides or hydrobromides. Preferred esters of the compounds of formula I are acyl or aroyl derivatives which will hydrolyse under physiological conditions.

The above compounds may be formulated to dosage forms using the principles which are known to those having average skill in the art. Suitable solvents, gel forming ingredients, dispersion agents, antioxidants and colourants may be added in a normal way.

It is preferred to administer the compounds enterally. When they are given orally, they may be in the form of tablets, granules, capsules, emulsions, suspensions or solutions. It is often advisable to use coated tablets or granules, i.e. so called enterocoated preparations, to ensure that the medicine reaches the desired part of the gastrointestinal tract. Alternatively, the compositions may be given rectally in the form of enemas or suppositories.

The effective dose varies considerably depending on location, degree and severity of the disease being treated as well as the age and the general condition of the patient. The effective dose is generally from about 20 to 1000 mg per day, preferably from 50 to 250 mg per day for an adult, once a day or divided into two to five doses.

### In vivo tests

The efficacy of the compounds can be demonstrated by using a model of chronic colonic inflammation in the rat, TNB-induced chronic colitis. TNB-induced chronic colonic inflammation in rats is an animal model which has been shown to resemble highly human IBD.

20 mg of TNB (2,4,6-trinitrobenzenesulfonic acid) in 50 % ethanol was administered into the lumen of the colon via a rubber catheter inserted rectally to Wistar rats. The test compounds were given rectally 1 h prior and 24, 48, 72 and 95 h after TNB. The rats were sacrificed 96 h after TNB and their colons were scored according to occurence of ulcers and inflammation. The number of animals in each group was at least 8. 5-ASA (5-aminosalicylic acid), a clinically used drug for the treatment of IBD was used as a reference compound. The results are presented in the Table 1.

**Table 1**

| The effect of test compounds on the damage score of chronic inflammation induced by TNB. | | |
|---|---|---|
| Test compound | Dose mg/kg | % inhibition against control |
| 5-ASA | 100 | 24 |
| 1 | 30 | 37 |
| 2 | 30 | 28 |
| 3 | 10 | 18 |
| | 30 | 57 |
| 4 | 30 | 54 |
| 5 | 30 | 43 |
| 6 | 30 | 41 |
| 7 | 10 | 60 |
| | 30 | 48 |
| 8 | 10 | 51 |
| | 30 | 62 |
| 9 | 30 | 40 |
| 10 | 10 | 27 |
| 11 | 30 | 32 |
| 12 | 30 | 52 |
| 13 | 30 | 58 |
| 14 | 30 | 30 |

### Test compounds

1 3-(4-hydroxyphenyl)methylene-2,4-pentanedione
2 3-(4-methoxyphenyl)methylene-2,4-pentanedione
3 3-(3-nitrophenyl)methylene-2,4-pentanedione
4 3-(4-trifluoromethylphenyl)methylene-2,4-pentanedione
5 3-(4-nitrophenyl)methylene-2,4-pentanedione
6 3-(2-nitrophenyl)methylene-2,4-pentanedione
7 3-(4-cyanophenyl)methylene-2,4-pentanedione
8 3-(3-cyanophenyl)methylene-2,4-pentanedione
9 3-[(4-(N-phenethyl)carboxamido-phenyl)methylene]-2,4-pentanedione
10 3-[(5-nitrofuryl)methylene]-2,4-pentanedione
11 4-[(4-cyanophenyl)methylene]-3,5-heptanedione
12 2-[(4-cyanophenyl)methylene]-1,3-dicyclopropyl-1,3-propanedione
13 2-[(3-trifluoromethylphenyl)methylene]-1,3-dicyclopropyl-1,3-propanedione
14 2-[(4-carboxyphenyl)methylene]-1,3-dicyclopropyl-1,3-propanedione

The test compounds significantly decreased the ulceration and inflammation of the colons. The test compounds were much more potent than the reference compound 5-ASA and should therefore be more effective than 5-ASA in the treatment of human IBD.

The following examples illustrate the production of compounds of use according to the invention.

### Example 1

### 3-[(4-Phenylsulfonylphenyl)methylene]-2,4-pentanedione

To a solution containing 2.1 g of 4-phenylsulfonylbenzaldehyde and 1.5 g of 2,4-pentanedione in 10 ml of 2-propanol was added 1 ml of thionyl chloride with stirring at 20°C. The solution was stirred for 1h at 20°C and evaporated to dryness in vacuo. The residue was crystallized from toluene, mp 102-105°C.

### Example 2

### 3-[(4-Methylsulfonylphenyl)methylene]-2,4-pentanedione

The procedure described in Example 1 was repeated by using 1.1 g of 4-methylsulfonylbenzaldehyde and 1.5 g of 2,4-pentanedione. The product was crystallized from ether, mp 139-140°C.

### Example 3

### 3-[(4-Carboxamidophenyl)methylene]-2,4-pentanedione

A mixture containing 1.8 g of 4-carboxamidobenzaldehyde, 1.1 g of 2,4-pentanedione, 0.2 g of piperidine and 0.2 g of acetic acid in 40 ml of toluene was refluxed for 3 h by using a Dean-Stark separator. The warm clean toluene solution was decanted from the tarry residue and cooled. The crystalline product was filtered and dried. Mp 154-155°C.

### Example 4

### 4-[(4-Nitrophenyl)methylene]-3,5-heptanedione

The procedure described in Example 1 was repeated by using 1.5 g of 4-nitrobenzaldehyde and 2.0 g of 3,5-heptanedione. Yield 1.7 g, yellow oil.

### Example 5

### 3-[(3,4-Dichlorophenyl)methylene]-2,4-pentanedione

The procedure described in Example 1 was repeated by using 4.26 g of 3,4-dichlorobenzaldehyde and 3.0 g of 2,4-pentanedione. Yield 1.3 g, mp 73°C.

### Example 6

### 3-[(4-Chloro-3-nitrophenyl)methylene]-2,4-pentanedione

The procedure described in Example 1 was repeated by using 1.85 g of 4-chloro-3-nitrobenzaldehyde and 1.5 g of 2,4-pentanedione. Yield 1.32 g, mp 110-111°C.

### Example 7

### 3-[(4-(N-Phenethyl)carboxamidophenyl)methylene]-2,4-pentanedione.

The procedure described in Example 1 was repeated by using 2.5 g of 4-(N-phenethyl)carboxamidobenzaldehyde and 1.0 g of 2,4-pentanedione. Yield 0,5 g, mp 146-149°C.

### Example 8

### 3-[(4-Formyl-3-nitrophenyl)methylene]-2,4-pentanedione

To a solution containing 1.84 g of 2-nitroterephtaldicarboxaldehyde and 1.00 g of 2,4-pentanedione in 10 ml trifluoroacetic acid were added 0.9 ml of thionyl chloride and a catalytic amount of water. The solution was stirred for 2 h at 20°C. 30 ml of water was added and the mixture was extracted with ether. The ether extract was washed several times with Na₂CO₃-solution and dried over K₂CO₃. After filtration the solvent was evaporated in vacuo. The product was purified by column chromatography. Yield 0.2 g, mp 102-104°C.

### Example 9

### 3-[(3,5-Dinitro-4-hydroxyphenyl)methylene]-2,4-pentanedione.

The procedure described in Example 1 was repeated by using 2.12 g of 3,5-dinitro-4-hydroxybenzaldehyde and 1.5 g of 2,4-pentanedione. Yield 1.9 g, mp 158°C.

### Example 10

### 3-[(3,5-Dinitrophenyl)methylene]-2,4-pentanedione

The procedure described in Example 1 was repeated by using 2.53 g 3,5-dinitrobenzaldehyde and 2.43 g of 2,4-pentanedione. Yield 0.8 g, mp 101-105°C.

### Example 11

### 3-[3-(4-Methoxyphenyl)propenylene]-2,4-pentanedione

To a solution containing 2.45 g of 4-methoxycinnamaldehyde in 90 ml of toluene was added 2.2 g of 2,4-pentanedione, 0,26 g of piperidine and 0.45 g of acetic acid. The solution was refluxed in N₂ atmosphere for 1 h and the water formed in the reaction was azeotropically distilled off by using a Dean-Stark water separator. The solution was evaporated to dryness in vacuo. The residue was trifurated with ether. The crystalls were filtered. Yield 0.65 g, mp 108-110°C.

### Example 12

### 3-[(5-Nitrofuryl)methylene]-2,4-pentanedione

A solution containing 2.43 g of 5-nitro-2-furanecarboxaldehyde diacetate, 1.5 g of 2,4-pentanedione and 0.1 g of water in 5 ml of tetrahydrofurane was saturated with hydrogen chloride gas at 20°C. The mixture was stirred for 1 h and evaporated to dryness in vacuo. The product was recrystallized from acetic acid. Yield 0.1 g, mp 117-120°C.

### Example 13

### 3-[(5-Nitrothienyl)methylene]-2,4-pentanedione

The procedure described in Example 12 was repeated by using 2,59 g of 5-nitro-2-thiophenecarboxaldehyde diacetate, 1.5 g of 2,4-pentanedione. Yield 0.5 g, yellow viscous oil.

### Example 14

### 3-(3-Pyridylmethylene)-2,4-pentanedione

The procedure described in Example 3 was repeated by using 3.21 g of pyridine-3-carboxaldehyde and 3.3 g of 2,4-pentanedione. Yield 2.95 g, mp 52-57°C.

### Example 15

### 3-(4-Pyridylmethylene)-2,4-pentanedione hydrochloride

A solution containing 1.1 g of pyridine-4-carboxaldehyde and 1.0 g of 2,4-pentanedione in 10 ml of N,N-dimethylformamide was saturated with hydrogen chloride gas at 20°C. The solution was stirred for 1 h at 20°C and then for 2 h at 80°C. The solvent was evaporated in vacuo and the residue was crystallized from ethanol. Yield 0.17 g, mp 175-186°C.

### Example 16

### 3-(3-Quinolylmethylene)-2,4-pentanedione hydrochloride

The procedure described in Example 3 was repeated by refluxing 2.4 g quinoline-3-aldehyde, 6.6 g 2,4-pentanedione, 0.4 g piperidine and 0.8 g acetic acid in 100 ml toluene. Yield 1.4 g, mp 99-101°C.

### Example 17

### 3-[(3-Carboxy-4-hydroxyphenyl)methylene]-2,4-pentanedione

A mixture containing 2 g 3-carboxy-4-hydroxybenzaldehyde, 4 ml 2,4-pentanedione and 20 ml tetrahydrofuran was saturated with hydrogen chloride gas. The solution was stirred for 20 h at 50°C and evaporated to dryness in vacuo. The residue was crystallized from ethyl ether, mp. 112-114°C.

### Example 18

### 3-[[(4-(2-Phenylethoxy)phenyl]methylene]-2,4-pentanedione

The procedure described in example 8 was repeated by using 5.9 g of 4-(2-phenylethoxy)benzaldehyde, 3.9 ml of 2,4-pentanedione, 26 ml of trifluoroacetic acid and 2.1 ml of thionyl chloride. Yield 4.7 g, an viscous oil.

### Example 19

### 2-[(4-Acetamidophenyl)methylene]-1,3-dicyclopropyl-1,3-propanedione

A mixture containing 1.6 g 4-acetamidobenzaldehyde, 1.5 g 1,3.dicyclopropyl-1,3-propanedione, 0.2 ml of piperidine and 0.4 ml of acetic acid in 50 ml of toluene was refluxed for 3 h by using a Dean-Stark separator. After standing over night at room temperature the crystalls were filtered and washed with toluene. The product was recrystallized from ethanol. Yield 2.0 g, mp 166-167°C.

### Example 20

### 2-[(4-Methoxyphenyl)methylene]-1,3-dicyclopropyl-1,3-propanedione

The procedure described in Example 19 was repeated by using 0.68 g 4-methoxybenzaldehyde and 0.77 g 1,3-dicyclopropyl-1,3-propanedione. Yield 0.46 g, mp 60.5-61.5°C.

### Example 21

### 4-[(3-Cyanophenyl)methylene]-3,5-heptanedione

The procedure described in Example 19 was repeated by using 1.82 g 3-cyanobenzaldehyde and 3.5 g 3,5-heptanedione. Yield 0.4 g, mp 51-52°C.

### Example 22

### 4-[(4-Cyanophenyl)methylene]-3,5-heptanedione

The produre described in Example 19 was repeated by using 2.6 g 4-cyanobenzaldehyde and 5.0 g 3,5-heptanedione. Yield 2.8 g, mp 59-60°C.

### Example 23

### 3-[(4-Cyanophenyl)methylene]-4-cyclopropyl-2,4-butanedione

The procedure described in Example 19 was repeated by using 2.6 g 4-cyanobenzaldehyde and 2.5 g 4-cyclopropyl-2,4-butanedione. The product was purified by column chromatography. Yield 0.37 g, mp 83-85°C.

### Example 24

### 2-[(4-Cyanophenyl)methylene]-1,3-dicyclopropyl-1,3-propanedione

The procedure described in Example 19 was repeated by using 3.78 g 4-cyanobenzaldehyde and 2.23 g 1,3-dicyclopropyl-1,3-propanedione. Yield 1.5 g, mp 100-102°C.

### Example 25

### 3-[(5-Bromothienyl)methylene]-2,4-pentanedione

To a solution containing 5.73 g 5-bromothiophene-2-carboxaldehyde and 5.0 g 2,4-pentanedione in 25 ml of 2-propanol, 2.9 ml thionylchloride was gradually added. The mixture was stirred for 2 h at 20°C. 120 ml of conc. Na₂SO₄-solution was then added and the mixture was extracted with ether. The solvent was evaporated in vacuo and the crystalline residue was washed with cold ether. Yield 3.9 g, mp 70-74°C.

### Example 26

### 2-[(3-Trifluoromethylphenyl)methylene]-1,3-dicyclopropyl-1,3-propanedione

The procedure described in Example 19 was repeated by using 2.15 ml 3-trifluoromethylbenzaldehyde and 2.42 g 1,3-dicyclopropyl-1,3-propanedione. Yield 2.71 g, mp 56-58°C.

### Example 27

### 2-[(4-Carboxyphenyl)methylene]-1,3-dicyclopropyl-1,3-propanedione

The procedure described in Example 19 was repeated by using 3.0 g 4-carboxybenzaldehyde and 3.06 g 1,3-dicyclopropyl-1,3-propanedione. Yield 1.7 g, mp 175-178°C.

## Claims (Claims for the following Contracting State(s): AT, BE, CH, LI, DE, DK, FR, GB, IT, LU, NL, SE)

1. Use of a compound of formula I 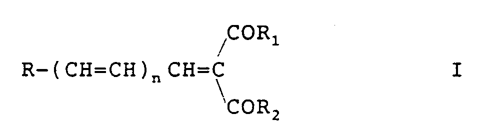 wherein n=0 or 1, R₁ and R₂ are independently methyl, ethyl or cyclopropyl and R is an optionally substituted phenyl or heteroaryl group; 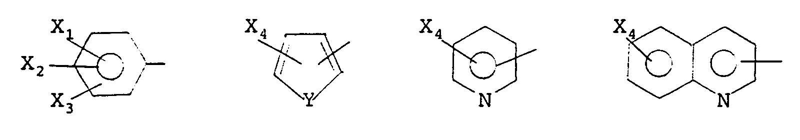 wherein X₁ is hydrogen, hydroxy or alkoxy which is optionally substituted by aryl; X₂ and X₃ are independently hydrogen, nitro, cyano, halo, trifluoromethyl, formyl, carboxy, acetamido or;
R₃S(O)ₘ
wherein m=0, 1 or 2 and R₃ is alkyl, aryl or aralkyl; or carbamoyl which is optionally substituted by alkyl, aryl or aralkyl; in which "alkyl" is an alkyl group having preferably 1 to 2 carbon atoms, "alkoxy" is an alkyl residue as defined above liked to an oxygen atom, and "aralkyl" is an alkyl group as defined above having aryl, preferably phenyl as the substituent; Y is oxygen or sulphur and X₄ is hydrogen, nitro or halo; or a pharmaceutically acceptable salt or ester thereof in the manufacture of a medicament for use in the treatment of inflammatory bowel disease.

2. The use according to claim 1, in which, in the compound of formula I, X₁ is hydroxy or alkoxy.

3. The use according to claim 1 or 2, in which, in the compound of formula I, one of X₂ and X₃ is nitro, cyano, trifluoromethyl, carboxy or carbamoyl which is substituted by aryl.

4. The use according to claims 1 to 3, in which the compound is
3-(4-hydroxyphenyl)methylene-2,4-pentanedione,
3-(4-methoxyphenyl)methylene-2,4-pentanedione,
3-(3-nitrophenyl)methylene-2,4-pentanedione,
3-(4-nitrophenyl)methylene-2,4-pentanedione,
3-(2-nitrophenyl)methylene-2,4-pentanedione,
3-[(4-(N-phenethyl)carboxamido-phenyl)methylene]-2,4-pentanedione,
3-[(5-nitrofuryl)methylene]-2,4-pentanedione, or
2-[(4-carboxyphenyl)methylene]-1,3-dicyclopropyl-1,3-propanedione or a pharmaceutically acceptable salt or ester thereof.

5. The use according to claims 1 or 3, in which, the compound is 3-(4-trifluoromethylphenyl)methylene-2,4-pentanedione.

6. The use according to claims 1 or 3, in which, the compound is 3-(4-cyanophenyl)methylene-2,4-pentanedione.

7. The use according to claims 1 or 3, in which, the compound is 3-(3-cyanophenyl)methylene-2,4-pentanedione.

8. The use according to claims 1 or 3, in which, the compound is 4-[(4-cyanophenyl)methylene]-3,5-heptanedione.

9. The use according to claims 1 or 3, in which, the compound is 2-[(4-cyanophenyl)methylene]-1,3-dicyclopropyl-1,3-propanedione.

10. This use according to claims 1 or 3, in which, the compound is 2-[(3-trifluoromethylphenyl)methylene]-1,3-di-cyclopropyl-1,3-propanedione.

11. A compound according to the general formula II 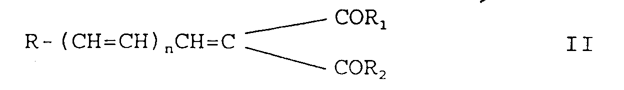 wherein n=0 or 1, R₁ is methyl, ethyl or cyclopropyl and R₂ is ethyl or cyclopropyl and R is 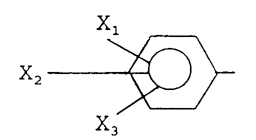 wherein X₁ is hydrogen, hydroxy or alkoxy which is optionally substituted by aryl; X₂ and X₃ are independently hydrogen, nitro, cyano, halo, trifluoromethyl, formyl, carboxy, acetamido, provided that when n=0, X₁, X₂ and X₃ are not all independently selected from the group consisting of hydrogen, hydroxy and nitro; or
R₃S(O)ₘ
wherein m=0, 1 or 2 and R₃ is alkyl, aryl or aralkyl; or carbamoyl which is optionally substituted by alkyl, aryl or aralkyl or R₁ and R₂ are methyl and R is 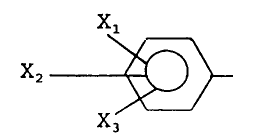 where X₁, X₂ and X₃ are as defined above provided that one of X₂ and X₃ is carbamoyl which is optionally substituted by alkyl, aryl or aralkyl, in which "alkyl", "alkoxy" and "aralkyl" are as defined herein; or a pharmaceutically acceptable salt or ester thereof, but excluding 4-(phenylmethylene)-3,5-heptanedione, 4-(3,4-dichlorophenylmethylene)-3,5-heptanedione and 3-(phenylmethylene)-2,4-hexanedione.

12. A compound according to claim 11 wherein in the compound of formula II one of X₂ and X₃ is cyano or trifluoromethyl.

13. 2-[(4-Acetamidophenyl)methylene]-1,3-dicyclopropyl-1,3-propanedione.

14. 2-[(4-Methoxyphenyl)methylene]-1,3-dicyclopropyl-1,3-propanedione.

15. 3-[(4-Cyanophenyl)methylene]-4-cyclopropyl-2,4-butanedione.

16. 2-[(4-Cyanophenyl)methylene]-1,3-dicyclopropyl-1,3-propanedione.

17. 2-[(3-Trifluoromethylphenyl)methylene]-1,3-dicyclopropyl-1,3-propanedione.

18. 2-[(4-Carboxyphenyl)methylene]-1,3-dicyclopropyl-1,3-propanedione or a pharmaceutically acceptable salt or ester thereof.

19. 4-[(4-Nitrophenyl)methylene]-3,5-heptanedione.

20. 4-[(3-Cyanophenyl)methylene]-3,5-heptanedione.

21. 4-[(4-Cyanophenyl)methylene]-3,5-heptanedione.

22. 3-[(4-Carboxamidophenyl)methylene]-2,4-pentanedione.

23. 3-[(4-(N-phenethyl)carboxamidophenyl)methylene]-2,4-pentanedione.

24. A compound according to the general formula III 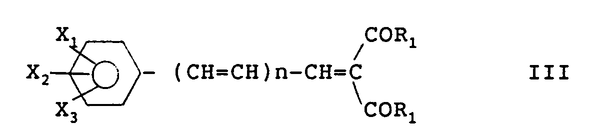 wherein R₁ is methyl, n is 0 or 1, X₁ is hydrogen, hydroxy or alkoxy which is optionally substituted by aryl, and X₂ is;
R₃S(O)ₘ
wherein m is 1 or 2 and R₃ is alkyl, aryl or aralkyl, and X₃ is hydrogen, nitro, cyano, halo, trifluoromethyl, formyl, carboxy or a group as defined for X₂; or X₂ and X₃ are both cyano; or X₂ is nitro and X₃ is formyl; or when X₁ is hydroxy, X₂ may be carboxy or X₂ and X₃ may both be hydrogen when n=0 and X₁ is alkoxy substituted by aryl, or when n=1 and X₁ is hydroxy or alkoxy optionally substituted by aryl; in which "alkyl" is an alkyl group having preferably 1 to 2 carbon atoms, "alkoxy" is an alkyl residue as defined above linked to an oxygen atom, and "aralkyl" is an alkyl group as defined above having aryl, preferably phenyl as the substituent; or a pharmaceutically acceptable salt or ester thereof.

25. 3-[(4-Phenylsulfonylphenyl)methylene]-2,4-pentanedione.

26. 3-[(4-Methylsulphonylphenyl)methylene]-2,4-pentanedione.

27. 3-[(4-Formyl-3-nitrophenyl)methylene]-2,4-pentanedione.

## Claims (Claims for the following Contracting State(s): ES, GR)

1. Use of a compound of formula I 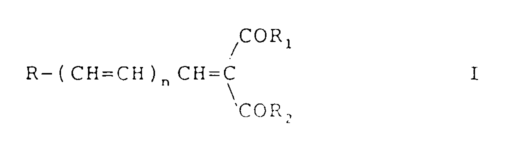 wherein n=0 or 1, R₁ and R₂ are independently methyl, ethyl or cyclopropyl and R is an optionally substituted phenyl or heteroaryl group; 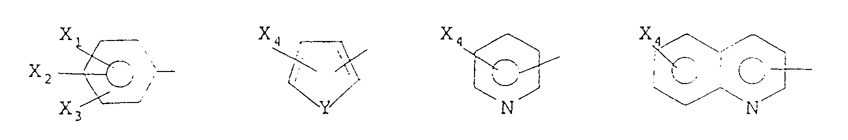 wherein X₁ is hydrogen, hydroxy or alkoxy which is optionally substituted by aryl; X₂ and X₃ are independently hydrogen, nitro, cyano, halo, trifluoromethyl, formyl, carboxy, acetamido or;
R₃S(O)ₘ
wherein m=0, 1 or 2 and R₃ is alkyl, aryl or aralkyl; or carbamoyl which is optionally substituted by alkyl, aryl or aralkyl; in which "alkyl" is an alkyl group having preferably 1 to 2 carbon atoms, "alkoxy" is an alkyl residue as defined above linked to an oxygen atom, and "aralkyl" is an alkyl group as defined above having aryl, preferably phenyl as the substituent; Y is oxygen or sulphur and X₄ is hydrogen, nitro or halo; or a pharmaceutically acceptable salt or ester thereof in the manufacture of a medicament for use in the treatment of inflammatory bowel disease.

2. The use according to claim 1, in which, in the compound of formula I, X₁ is hydroxy or alkoxy.

3. The use according to claim 1 or 2, in which, in the compound formula I, one of X₂ and X₃ is nitro, cyano, trifluoromethyl, carboxy or carbamoyl which is substituted by aryl.

4. The use according to claims 1 to 3, in which the compound is
3-(4-hydroxyphenyl)methylene-2,4-pentanedione,
3-(4-methoxyphenyl)methylene-2,4-pentanedione,
3-(3-nitrophenyl)methylene-2,4-pentanedione,
3-(4-nitrophenyl)methylene-2,4-pentanedione,
3-(2-nitrophenyl)methylene-2,4-pentanedione,
3-[(4-(N-phenethyl)carboxamido-phenyl)methylene]-2,4-pentanedione,
3-[(5-nitrofuryl)methylene]-2,4-pentanedione, or
2-[(4-carboxyphenyl)methylene]-1,3-dicyclopropyl-1,3-propanedione or a pharmaceutically acceptable salt or ester thereof.

5. The use according to claims 1 or 3, in which, the compound is 3-(4-trifluoromethylphenyl)methylene-2,4-pentanedione.

6. The use according to claims 1 or 3, in which, the compound is 3-(4-cyanophenyl)methylene-2,4-pentanedione.

7. The use according to claims 1 or 3, in which, the compound is 3-(3-cyanophenyl)methylene-2,4-pentanedione.

8. The use according to claims 1 or 3, in which, the compound is 4-[(4-cyanophenyl)methylene]-3,5-heptanedione.

9. The use according to claims 1 or 3, in which, the compound is 2-[(4-cyanophenyl)methylene]-1,3-dicyclopropyl-1,3-propanedione.

10. This use according to claims 1 or 3, in which, the compound is 2-[(3-trifluoromethylphenyl)methylene]-1,3-dicyclopropyl-1,3-propanedione.

11. A process for the preparation of a compound according to the general formula II 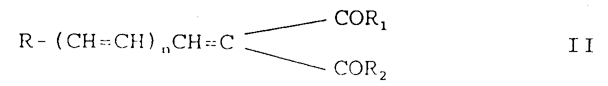 wherein n=0 or 1, R₁ is methyl, ethyl or cyclopropyl and R₂ is ethyl or cyclopropyl and R is 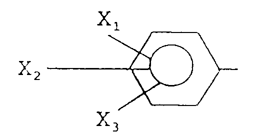 wherein X₁ is hydrogen, hydroxy or alkoxy which is optionally substituted by aryl; X₂ and X₃ are independently hydrogen, nitro, cyano, halo, trifluoromethyl, formyl, carboxy, acetamido, provided that when n=0, X₁, X₂ and X₃ are not all independently selected from the group consisting of hydrogen, hydroxy and nitro; or
R₃S(O)ₘ
wherein m=0, 1 or 2 and R₃ is alkyl, aryl or aralkyl; or carbamoyl which is optionally substituted by alkyl, aryl or aralkyl or R₁ and R₂ are methyl and R is 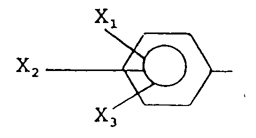 where X₁, X₂ and X₃ are as defined above provided that one of X₂ and X₃ is carbamoyl which is optionally substituted by alkyl, aryl or aralkyl, in which "alkyl", "alkoxy" and "aralkyl" are as defined herein; or a pharmaceutically acceptable salt or ester thereof, but excluding 4-(phenylmethylene)-3,5-heptanedione, 4-(3,4-dichlorophenylmethylene)-3,5-heptanedione and 3-(phenylmethylene)-2,4-hexanedione, said process comprising reacting a compound of formula IV
R₁-CO-CH₂-CO-R₂ IV
in which R₁ and R₂ are as defined above with a compound of formula V
R-(CH=CH)ₙ-Z V
in which n and R are as defined above and Z is CHO or -CH₂-Q, wherein Q is halogen or some other activated group, in the presence of acidic or basic catalyst to produce a compound of formula II or a compound of formula VI 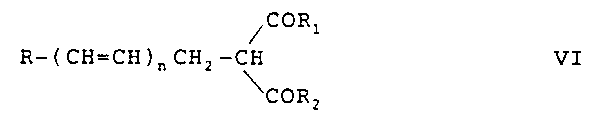 in which n, R₁, R₂ and R are as defined above, which compound is halogenated to give a compound of the formula VII 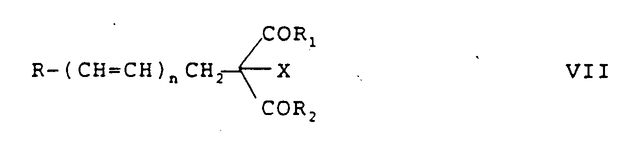 in which n, R₁, R₂ and R are as defined above and X is halogen and dehydrohalogenating compound VII to produce a compound of the formula II.

12. A process according to claim 11 wherein in the compound of formula II one of X₂ and X₃ is cyano or trifluoromethyl.

13. A process according to claim 11 for the preparation of 2-[(4-Acetamidophenyl)methylene]-1,3-dicyclopropyl-1,3-propanedione.

14. A process according to claim 11 for the preparation of 2-[(4-Methoxyphenyl)methylene]-1,3-dicyclopropyl-1,3-propanedione.

15. A process according to claim 11 for the preparation of 3-[(4-Cyanophenyl)methylene]-4-cyclopropyl-2,4-butanedione.

16. A process according to claim 11 for the preparation of 2-[(4-Cyanophenyl)methylene]-1,3-dicyclopropyl-1,3-propanedione.

17. A process according to claim 11 for the preparation of 2-[(3-Trifluoromethylphenyl)methylene]-1,3-dicyclopropyl-1,3-propanedione.

18. A process according to claim 11 for, the preparation of 2-[(4-Carboxyphenyl)methylene]-1,3-dicyclopropyl-1,3-propanedione or a pharmaceutically acceptable salt or ester thereof.

19. A process according to claim 11 for the preparation of 4-[(4-Nitrophenyl)methylene]-3,5-heptanedione.

20. A process according to claim 11 for the preparation of 4-[(3-Cyanophenyl)methylene]-3,5-heptanedione.

21. A process according to claim 11 for the preparation of 4-[(4-Cyanophenyl)methylene]-3,5-heptanedione.

22. A process according to claim 11 for the preparation of 3-[(4-Carboxamidophenyl)methylene]-2,4-pentanedione.

23. A process according to claim 11 for the preparation of 3-[(4-(N-phenethyl)carboyamidophenyl)methylene]-2,4-pentanedione.

24. A process for the preparation of a compound according to the general formula III 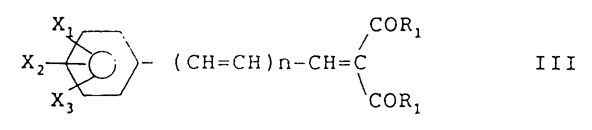 wherein R₁ is methyl, n is 0 or 1, X₁ is hydrogen, hydroxy or alkoxy which is optionally substituted by aryl, and X₂ is;
R₃S(O)ₘ
wherein m is 1 or 2 and R₃ is alkyl, aryl or aralkyl, and X₃ is hydrogen, nitro, cyano, halo, trifluoromethyl, formyl, carboxy or a group as defined for X₂ ; or X₂ and X₃ are both cyano; or X₂ is nitro and X₃ is formyl; or when X₁ is hydroxy, X₂ may be carboxy or X₂ and X₃ may both be hydrogen when n=0 and X₁ is alkoxy substituted by aryl, or when n=1 and X₁ is hydroxy or alkoxy optionally substituted by aryl; in which "alkyl" is an alkyl group having preferably 1 to 2 carbon atoms, "alkoxy" is an alkyl residue as defined above linked to an oxygen atom, and "aralkyl" is an alkyl group as defined above having aryl, preferably phenyl as the substituent; or a pharmaceutically acceptable salt or ester thereof, said process comprising reacting a compound of the formula IV
R₁-CO-CH₂-CO-R₂ IV
in which R₁ and R₂ are as defined above with a compound of formula V
R-(CH=CH)ₙ-Z V
in which n and R are as defined above and Z is CHO or -CH₂-Q, wherein Q is halogen or some other activated group, in the presence of acidic or basic catalyst to produce a compound of formula III or a compound of formula VI 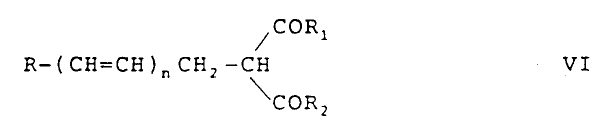 in which n, R₁, and R₂ are as defined above and R is phenyl substituted by X₁, X₂ and X₃ as defined above, which compound is halogenated to give a compound of the formula VII 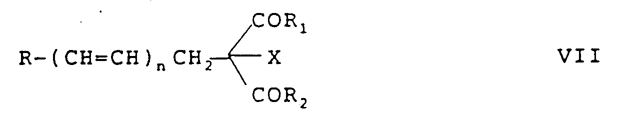 in which n, R₁ , R₂ and R are as defined above and X is halogen and dehydrohalogenating compound VII to produce a compound of the formula III.

25. Process according to claim 24 for the preparation of 3-[(4-Phenylsulfonylphenyl)methylene]-2,4-pentanedione.

26. Process according to claim 24 for the preparation of 3-[(4-Methylsulphonylphenyl)methylene]-2,4-pentanedione.

27. Process according to claim 24 for the preparation of 3-[(4-Formyl-3-nitrophenyl)methylene]-2,4-pentanedione.

28. A compound according to the general formula II 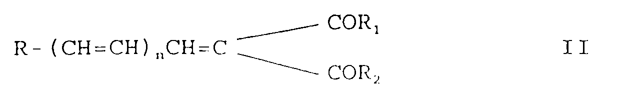 wherein n=0 or 1, R₁ is methyl, ethyl or cyclopropyl and R₂ is ethyl or cyclopropyl and R is 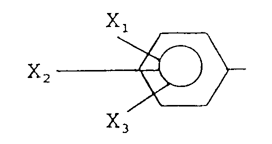 wherein X₁ is hydrogen, hydroxy or alkoxy which is optionally substituted by aryl; X₂ and X₃ are independently hydrogen, nitro, cyano, halo, trifluoromethyl, formyl, carboxy, acetamido, provided that when n=0, X₁, X₂ and X₃ are not all independently selected from the group consisting of hydrogen, hydroxy and nitro; or
R₃S(O)ₘ
wherein m=0, 1 or 2 and R₃ is alkyl, aryl or aralkyl; or carbamoyl which is optionally substituted by alkyl, aryl or aralkyl or R₁ and R₂ are methyl and R is 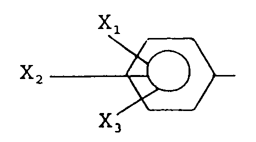 where X₁, X₂ and X₃ are as defined above provided that one of X₂ and X₃ is carbamoyl which is optionally substituted by alkyl, aryl or aralkyl, in which "alkyl", "alkoxy" and "aralkyl" are as defined herein; or a pharmaceutically acceptable salt or ester thereof, but excluding 4-(phenylmethylene)-3,5-heptanedione, 4-(3,4-dichlorophenylmethylene)-3,5-heptanedione and 3-(phenylmethylene)-2,4-hexanedione.

29. A compound according to the general formula III 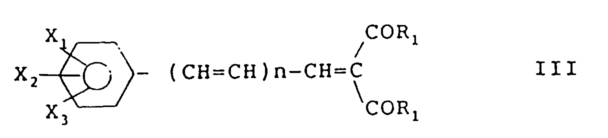 wherein R₁ is methyl, n is 0 or 1, X₁ is hydrogen, hydroxy or alkoxy which is optionally substituted by aryl, and X₂ is;
R₃S(O)ₘ
wherein m is 1 or 2 and R₃ is alkyl, aryl or aralkyl, and X₃ is hydrogen, nitro, cyano, halo, trifluoromethyl, formyl, carboxy or a group as defined for X₂; or X₂ and X₃ are both cyano; or X₂ is nitro and X₃ is formyl; or when X₁ is hydroxy, X₂ may be carboxy or X₂ and X₃ may both be hydrogen when n=0 and X₁ is alkoxy substituted by aryl, or when n=1 and X₁ is hydroxy or alkoxy optionally substituted by aryl; in which "alkyl" is an alkyl group having preferably 1 to 2 carbon atoms, "alkoxy" is an alkyl residue as defined above linked to an oxygen atom, and "aralkyl" is an alkyl group as defined above having aryl, preferably phenyl as the substituent; or a pharmaceutically acceptable salt or ester thereof.

30. 3-[(4-Methylsulphonylphenyl)methylene]-2,4-pentanedione

## Patentansprüche (Patentansprüche für folgende(n) Vertragsstaat(en): AT, BE, CH, LI, DE, DK, FR, GB, IT, LU, NL, SE)

1. Verwendung einer Verbindung der Formel I 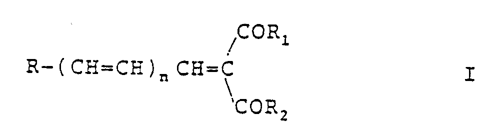 worin n=0 oder 1 ist, R₁ und R₂ unabhängig voneinander Methyl, Ethyl oder Cyclopropyl bedeuten und R eine gegebenenfalls substituierte Phenyl- oder Heteroarylgruppe 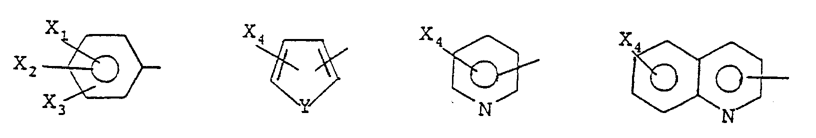 bedeutet, worin X₁ Wasserstoff, Hydroxy oder gegebenenfalls mit Aryl substituiertes Alkoxy bedeutet; X₂ und X₃ unabhängig voneinander Wasserstoff, Nitro, Cyano, Halogen, Trifluormethyl, Formyl, Carboxy, Acetamido oder
R₃S(O)ₘ
worin m=0, 1 oder 2 ist und R₃ Alkyl, Aryl oder Aralkyl bedeutet; oder gegebenenfalls mit Alkyl, Aryl oder Aralkyl substituiertes Carbamoyl bedeuten; wobei "Alkyl" eine Alkylgruppe mit vorzugsweise 1 bis 2 Kohlenstoffatomen bedeutet, "Alkoxy" einen wie oben definierten Alkylrest bedeutet, der an ein Sauerstoffatom gebunden ist, und "Aralkyl" eine wie oben definierte Alkylgruppe mit Aryl, vorzugsweise Phenyl, als Substituent bedeutet; Y Sauerstoff oder Schwefel bedeutet und X₄ Wasserstoff, Nitro oder Halogen bedeutet; oder eines pharmazeutisch verträglichen Salzes oder Esters davon, zur Herstellung eines Medikaments zur Behandlung entzündlicher Darmerkrankungen.

2. Verwendung gemäß Anspruch 1, worin X₁ in der Verbindung der Formel I Hydroxy oder Alkoxy ist.

3. Verwendung gemäß Anspruch 1 oder 2, worin einer der Reste X₂ und X₃ in der Verbindung der Formel I Nitro, Cyano, Trifluormethyl, Carboxy oder mit Aryl substituiertes Carbamoyl ist.

4. Verwendung gemäß den Ansprüchen 1 bis 3, worin die Verbindung
3-(4-Hydroxyphenyl)methylen-2,4-pentandion,
3-(4-Methoxyphenyl)methylen-2,4-pentandion,
3-(3-Nitrophenyl)methylen-2,4-pentandion,
3-(4-Nitrophenyl)methylen-2,4-pentandion,
3-(2-Nitrophenyl)methylen-2,4-pentandion,
3-[(4-(N-Phenethyl)carboxamidophenyl)methylen]-2,4-pentandion,
3-[(5-Nitrofuryl)methylen]-2,4-pentandion, oder
2-[(4-Carboxyphenyl)methylen]-1,3-dicyclopropyl-1,3-propandion,
oder ein pharmazeutisch verträgliches Salz oder ein pharmazeutisch verträglicher Ester davon ist.

5. Verwendung gemäß den Ansprüchen 1 oder 3, worin die Verbindung 3-(4-Trifluormethylphenyl)methylen-2,4-pentandion ist.

6. Verwendung gemäß den Ansprüchen 1 oder 3, worin die Verbindung 3-(4-Cyanophenyl)methylen-2,4-pentandion ist.

7. Verwendung gemäß den Ansprüchen 1 oder 3, worin die Verbindung 3-(3-Cyanophenyl)methylen-2,4-pentandion ist.

8. Verwendung gemäß den Ansprüchen 1 oder 3, worin die Verbindung 4-[(4-Cyanophenyl)methylen]-3,5-heptandion ist.

9. Verwendung gemäß den Ansprüchen 1 oder 3, worin die Verbindung 2-[(4-Cyanophenyl)methylen]-1,3-dicyclo-propyl-1,3-propandion ist.

10. Verwendung gemäß den Ansprüchen 1 oder 3, worin die Verbindung 2-[(3-Trifluormethylphenyl)methylen]-1,3-dicyclopropyl-1,3-propandion ist.

11. Verbindung der allgemeinen Formel II 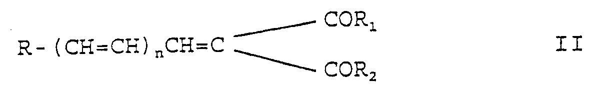 worin n=0 oder 1 ist, R₁ Methyl, Ethyl oder Cyclopropyl bedeutet und R₂ Ethyl oder Cyclopropyl bedeutet und R 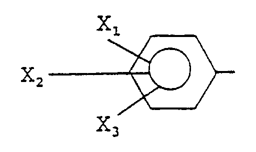 bedeutet, worin X₁ Wasserstoff, Hydroxy oder gegebenenfalls mit Aryl substituiertes Alkoxy bedeutet; X₂ und X₃ unabhängig voneinander Wasserstoff, Nitro, Cyano, Halogen, Trifluormethyl, Formyl, Carboxy, Acetamido bedeuten, mit der Einschränkung, daß wenn n=0 ist, X₁, X₂ und X₃ nicht alle unabhängig ausgewählt sind aus der Gruppe bestehend aus Wasserstoff, Hydroxy und Nitro; oder
R₃S(O)ₘ
worin m=0, 1 oder 2 ist und R₃ Alkyl, Aryl oder Aralkyl bedeutet, oder gegebenenfalls mit Alkyl, Aryl oder Aralkyl substituiertes Carbamoyl bedeutet, oder R₁ und R₂ Methyl bedeuten und R 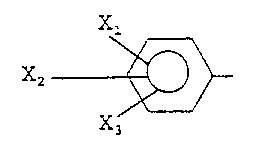 bedeutet, worin X₁, X₂ und X₃ wie oben definiert sind, mit der Einschränkung, daß einer der Reste X₂ und X₃ gegebenenfalls mit Alkyl, Aryl oder Aralkyl substituiertes Carbamoyl ist, wobei "Alkyl", "Alkoxy" und "Aralkyl" wie oben definiert sind; oder ein pharmazeutisch verträgliches Salz oder ein pharmazeutisch verträglicher Ester davon, ausgenommen jedoch
4-(Phenylmethylen)-3,5-heptandion,
4-(3,4-Dichlorphenylmethylen)-3,5-heptandion und
3-(Phenylmethylen)-2,4-hexandion.

12. Verbindung gemäß Anspruch 11, worin einer der Reste X₂ und X₃ in der Verbindung der Formel II Cyano oder Trifluormethyl ist.

13. 2-[(4-Acetamidophenyl)methylen]-1,3-dicyclopropyl-1,3-propandion.

14. 2-[(4-Methoxyphenyl)methylen]-1,3-dicyclopropyl-1,3 propandion.

15. 3-[(4-Cyanophenyl)methylen]-4-cyclopropyl-2,4-butandion.

16. 2-[(4-Cyanophenyl)methylen]-1,3-dicyclopropyl-1,3-propandion.

17. 2-[(3-Trifluormethylphenyl)methylen]-1,3-dicyclopropyl-1,3-propandion.

18. 2-[(4-Carboxyphenyl)methylen]-1,3-dicyclopropyl-1,3-propandion oder ein pharmazeutisch verträgliches Salz oder ein pharmazeutisch verträglicher Ester davon.

19. 4-[(4-Nitrophenyl)methylen]-3,5-heptandion

20. 4-[(3-Cyanophenyl)methylen]-3,5-heptandion.

21. 4-[(4-Cyanophenyl)methylen]-3,5-heptandion.

22. 3-[(4-Carboxamidophenyl)methylen]-2,4-pentandion.

23. 3-[(4-(N-Phenethyl)carboxamidophenyl)methylen]-2,4-pentandion.

24. Verbindung gemäß der allgemeinen Formel III 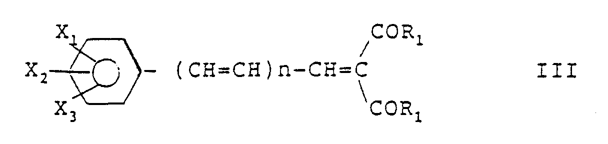 worin R₁ Methyl bedeutet, n 0 oder 1 ist, X₁ Wasserstoff, Hydroxy oder gegebenenfalls mit Aryl substituiertes Alkoxy bedeutet, und X₂
R₃S(O)ₘ
bedeutet, worin m 1 oder 2 ist und R₃ Alkyl, Aryl oder Aralkyl ist, und X₃ Wasserstoff, Nitro, Cyano, Halogen, Trifluormethyl, Formyl, Carboxy oder eine wie für X₂ definierte Gruppe bedeutet; oder X₂ und X₃ beide Cyano sind; oder X₂ Nitro und X₃ Formyl ist; oder worin wenn X₁ Hydroxy ist, X₂ Carboxy sein kann oder X₂ und X₃ beide Wasserstoff sein können, wenn n=0 und X₁ mit Aryl substituiertes Alkoxy ist oder wenn n=1 ist und X₁ Hydroxy oder gegebenenfalls mit Aryl substituiertes Alkoxy ist; wobei "Alkyl" eine Alkylgruppe mit vorzugsweise 1 bis 2 Kohlenstoffatomen bedeutet, "Alkoxy" einen wie oben definierten Alkylrest bedeutet, der an ein Sauerstoffatom gebunden ist, und "Aralkyl" eine wie oben definierte Alkylgruppe mit Aryl, vorzugsweise Phenyl, als Substituent bedeutet; oder ein pharmazeutisch verträgliches Salz oder ein pharmazeutisch verträglicher Ester davon.

25. 3-[(4-Phenylsulfonylphenyl)methylen]-2,4-pentandion.

26. 3-[(4-Methylsulfonylphenyl)methylen]-2,4-pentandion.

27. 3-[(4-Formyl-3-nitrophenyl)methylen]-2,4-pentandion.

## Patentansprüche (Patentansprüche für folgende(n) Vertragsstaat(en): ES, GR)

1. Verwendung einer Verbindung der Formel I 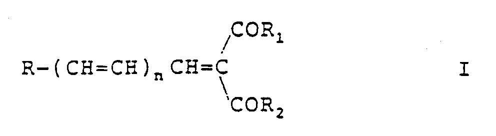 worin n=0 oder 1 ist, R₁ und R₂ unabhängig voneinander Methyl, Ethyl oder Cyclopropyl bedeuten und R eine gegebenenfalls substituierte Phenyl- oder Heteroarylgruppe 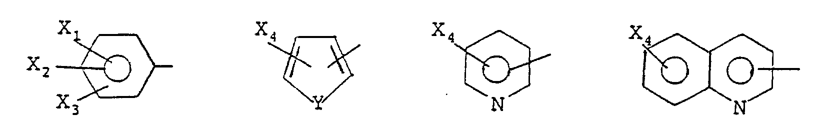 bedeutet, worin X₁ Wasserstoff, Hydroxy oder gegebenenfalls mit Aryl substituiertes Alkoxy bedeutet; X₂ und X₃ unabhängig voneinander Wasserstoff, Nitro, Cyano, Halogen, Trifluormethyl, Formyl, Carboxy, Acetamido oder
R₃S(O)ₘ
worin m=0, 1 oder 2 ist und R₃ Alkyl, Aryl oder Aralkyl bedeutet; oder gegebenenfalls mit Alkyl, Aryl oder Aralkyl substituiertes Carbamoyl bedeuten; wobei "Alkyl" eine Alkylgruppe mit vorzugsweise 1 bis 2 Kohlenstoffatomen bedeutet, "Alkoxy" einen wie oben definierten Alkylrest bedeutet, der an ein Sauerstoffatom gebunden ist, und "Aralkyl" eine wie oben definierte Alkylgruppe mit Aryl, vorzugsweise Phenyl, als Substituent bedeutet; Y Sauerstoff oder Schwefel bedeutet und X₄ Wasserstoff, Nitro oder Halogen bedeutet; oder eines pharmazeutisch verträglichen Salzes oder Esters davon, zur Herstellung eines Medikaments zur Behandlung entzündlicher Darmerkrankungen.

2. Verwendung gemäß Anspruch 1, worin X₁ in der Verbindung der Formel I Hydroxy oder Alkoxy ist.

3. Verwendung gemäß Anspruch 1 oder 2, worin einer der Reste X₂ und X₃ in der Verbindung der Formel I Nitro, Cyano, Trifluormethyl, Carboxy oder mit Aryl substituiertes Carbamoyl ist.

4. Verwendung gemäß den Ansprüchen 1 bis 3, worin die Verbindung
3-(4-Hydroxyphenyl)methylen-2,4-pentandion,
3-(4-Methoxyphenyl)methylen-2,4-pentandion,
3-(3-Nitrophenyl)methylen-2,4-pentandion,
3-(4-Nitrophenyl)methylen-2,4-pentandion,
3-(2-Nitrophenyl)methylen-2,4-pentandion,
3-[(4-(N-Phenethyl)carboxamidophenyl)methylen]-2,4-pentandion,
3-[(5-Nitrofuryl)methylen]-2,4-pentandion, oder
2-[(4-Carboxyphenyl)methylen]-1,3-dicyclopropyl-1,3-propandion,
oder ein pharmazeutisch verträgliches Salz oder ein pharmazeutisch verträglicher Ester davon ist.

5. Verwendung gemäß den Ansprüchen 1 oder 3, worin die Verbindung 3-(4-Trifluormethylphenyl)methylen-2,4-pentandion ist.

6. Verwendung gemäß den Ansprüchen 1 oder 3, worin die Verbindung 3-(4-Cyanophenyl)methylen-2,4-pentandion ist.

7. Verwendung gemäß den Ansprüchen 1 oder 3, worin die Verbindung 3-(3-Cyanophenyl)methylen-2,4-pentandion ist.

8. Verwendung gemäß den Ansprüchen 1 oder 3, worin die Verbindung 4-[(4-Cyanophenyl)methylen]-3,5-heptandion ist.

9. Verwendung gemäß den Ansprüchen 1 oder 3, worin die Verbindung 2-[(4-Cyanophenyl)methylen]-1,3-dicyclo-propyl-1,3-propandion ist.

10. Verwendung gemäß den Ansprüchen 1 oder 3, worin die Verbindung 2-[(3-Trifluormethylphenyl)methylen]-1,3-dicyclopropyl-1,3-propandion ist.

11. Verfahren zur Herstellung einer Verbindung der allgemeinen Formel II 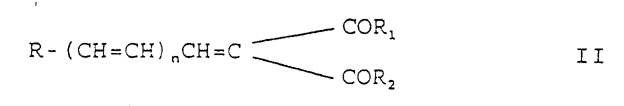 worin n=0 oder 1 ist, R₁ Methyl, Ethyl oder Cyclopropyl bedeutet und R₂ Ethyl oder Cyclopropyl bedeutet und R 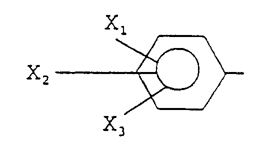 bedeutet, worin X₁ Wasserstoff, Hydroxy oder gegebenenfalls mit Aryl substituiertes Alkoxy bedeutet; X₂ und X₃ unabhängig voneinander Wasserstoff, Nitro, Cyano, Halogen, Trifluormethyl, Formyl, Carboxy, Acetamido bedeuten, mit der Einschränkung, daß wenn n=0 ist, X₁, X₂ und X₃ nicht alle unabhängig ausgewählt sind aus der Gruppe bestehend aus Wasserstoff, Hydroxy und Nitro; oder
R₃S(O)ₘ
worin m=0, 1 oder 2 ist und R₃ Alkyl, Aryl oder Aralkyl bedeutet, oder gegebenenfalls mit Alkyl, Aryl oder Aralkyl substituiertes Carbamoyl bedeutet, oder R₁ und R₂ Methyl bedeuten und R 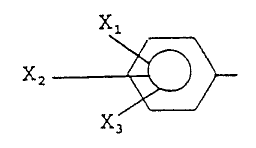 bedeutet, worin X₁, X₂ und X₃ wie oben definiert sind, mit der Einschränkung, daß einer der Reste X₂ und X₃ gegebenenfalls mit Alkyl, Aryl oder Aralkyl substituiertes Carbamoyl ist, wobei "Alkyl", "Alkoxy" und "Aralkyl" wie oben definiert sind; oder eines pharmazeutisch verträglichen Salzes oder eines pharmazeutisch verträglichen Esters davon, ausgenommen
4-(Phenylmethylen)-3,5-heptandion,
4-(3,4-Dichlorphenylmethylen)-3,5-heptandion und
3-(Phenylmethylen)-2,4-hexandion,
umfassend die Umsetzung einer Verbindung der Formel IV
R₁-CO-CH₂-CO-R₂ IV
worin R₁ und R₂ wie oben definiert sind, mit einer Verbindung der Formel V
R-(CH=CH)ₙ-Z V
worin n und R wie oben definiert sind und Z CHO oder -CH₂-Q bedeutet, worin Q Halogen oder irgendeine andere aktivierte Gruppe ist, in Gegenwart eines sauren oder basischen Katalysators zu einer Verbindung der Formel II oder einer Verbindung der Formel VI 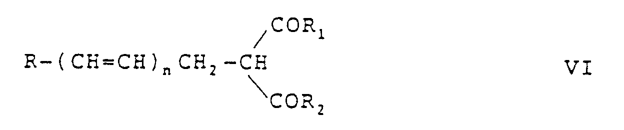 worin n, R₁, R₂ und R wie oben definiert sind, welche Verbindung zu einer Verbindung der Formel VII 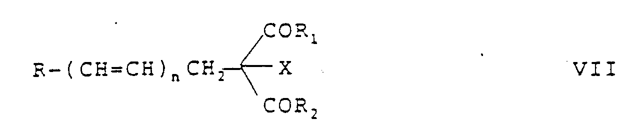 worin R₁ und R₂ wie oben definiert sind und X Halogen ist, halogeniert wird, und die Dehydrohalogenierung von Verbindung VII zu einer Verbindung der Formel II.

12. Verfahren gemäß Anspruch 11, worin einer der Reste X₂ und X₃ in der Verbindung der Formel II Cyano oder Trifluormethyl ist.

13. Verfahren gemäß Anspruch 11 zur Herstellung von 2-[(4-Acetamidophenyl)methylen]-1,3-dicyclopropyl-1,3-propandion.

14. Verfahren gemäß Anspruch 11 zur Herstellung von 2-[(4-Methoxyphenyl)methylen]-1,3-dicyclopropyl-1,3-propandion.

15. Verfahren gemäß Anspruch 11 zur Herstellung von 3-[(4-Cyanophenyl)methylen]-4-cyclopropyl-2,4-butandion.

16. Verfahren gemäß Anspruch 11 zur Herstellung von 2-[(4-Cyanophenyl)methylen]-1,3-dicyclopropyl-1,3-propandion.

17. Verfahren gemäß Anspruch 11 zur Herstellung von 2-[(3-Trifluormethylphenyl)methylen]-1,3-dicyclopropyl-1,3-propandion.

18. Verfahren gemäß Anspruch 11 zur Herstellung von 2-[(4-Carboxyphenyl)methylen]-1,3-dicyclopropyl-1,3-propandion oder eines pharmazeutisch verträglichen Salzes oder eines pharmazeutisch verträglichen Esters davon.

19. Verfahren gemäß Anspruch 11 zur Herstellung von 4-[(4-Nitrophenyl)methylen]-3,5-heptandion.

20. Verfahren gemäß Anspruch 11 zur Herstellung von 4-[(3-Cyanophenyl)methylen]-3,5-heptandion.

21. Verfahren gemäß Anspruch 11 zur Herstellung von 4-[(4-Cyanophenyl)methylen]-3,5-heptandion.

22. Verfahren gemäß Anspruch 11 zur Herstellung von 3-[(4-Carboxamidophenyl)methylen]-2,4-pentandion.

23. Verfahren gemäß Anspruch 11 zur Herstellung von 3-[(4-(N-Phenethyl)carboxamidophenyl)methylen]-2,4-pentandion.

24. Verfahren zur Herstellung einer Verbindung gemäß der allgemeinen Formel III 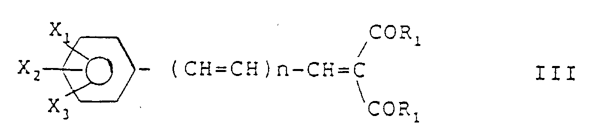 worin R₁ Methyl bedeutet, n 0 oder 1 ist, X₁ Wasserstoff, Hydroxy oder gegebenenfalls mit Aryl substituiertes Alkoxy bedeutet, und X₂
R₃S(O)ₘ
bedeutet, worin m 1 oder 2 ist und R₃ Alkyl, Aryl oder Aralkyl ist, und X₃ Wasserstoff, Nitro, Cyano, Halogen, Trifluormethyl, Formyl, Carboxy oder eine wie für X₂ definierte Gruppe bedeutet; oder X₂ und X₃ beide Cyano sind; oder X₂ Nitro und X₃ Formyl ist; oder worin wenn X₁ Hydroxy ist, X₂ Carboxy sein kann oder X₂ und X₃ beide Wasserstoff sein können, wenn n=0 und X₁ mit Aryl substituiertes Alkoxy ist oder wenn n=1 und X₁ Hydroxy oder gegebenenfalls mit Aryl substituiertes Alkoxy ist; wobei "Alkyl" eine Alkylgruppe mit vorzugsweise 1 bis 2 Kohlenstoffatomen bedeutet, "Alkoxy" einen wie oben definierten Alkylrest bedeutet, der an ein Sauerstoffatom gebunden ist, und "Aralkyl" eine wie oben definierte Alkylgruppe mit Aryl, vorzugsweise Phenyl, als Substituent bedeutet; oder eines pharmazeutisch verträglichen Salzes oder eines pharmazeutisch verträglichen Esters davon, umfassend die Umsetzung einer Verbindung der Formel IV
R₁-CO-CH₂-CO-R₂ IV
worin R₁ und R₂ wie oben definiert sind, mit einer Verbindung der Formel V
R-(CH=CH)ₙ-Z V
worin n und R wies oben definiert sind und Z CHO oder -CH₂-Q bedeutet, worin Q Halogen oder irgendeine andere aktivierte Gruppe ist, in Gegenwart eines sauren oder basischen Katalysators zu einer Verbindung der Formel III oder einer Verbindung der Formel VI 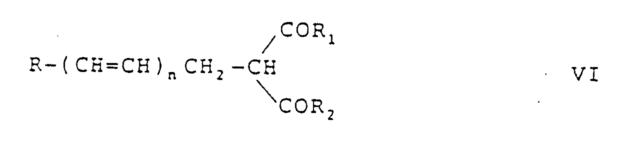 worin n, R₁ und R₂ wie oben definiert sind und R Phenyl bedeutet, das mit X₁, X₂ und X₃, die wie oben definuiert sind, substituiert ist, welche Verbindung zu einer Verbindung der Formel VII 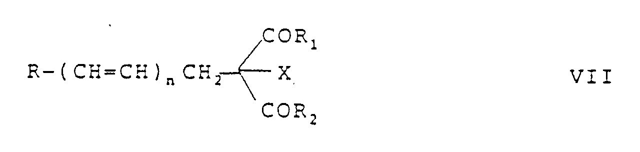 worin n, R₁, R₂ und R wie oben definiert sind und X Halogen ist, halogeniert wird, und die Dehydrohalogenierung von Verbindung VII zu einer Verbindung der Formel III.

25. Verfahren gemäß Anspruch 24 zur Herstellung von 3-[(4-Phenylsulfonylphenyl)methylen]-2,4-pentandion.

26. Verfahren gemäß Anspruch 24 zur Herstellung von 3-[(4-Methylsulfonylphenyl)methylen]-2,4-pentandion.

27. Verfahren gemäß Anspruch 24 zur Herstellung von 3-[(4-Formyl-3-nitrophenyl)methylen]-2,4-pentandion.

28. Verbindung der allgemeinen Formel II 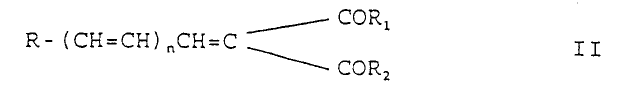 worin n=0 oder 1 ist, R₁ Methyl, Ethyl oder Cyclopropyl bedeutet und R₂ Ethyl oder Cyclopropyl bedeutet und R 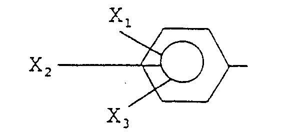 bedeutet, worin X₁ Wasserstoff, Hydroxy oder gegebenenfalls mit Aryl substituiertes Alkoxy bedeutet; X₂ und X₃ unabhängig voneinander Wasserstoff, Nitro, Cyano, Halogen, Trifluormethyl, Formyl, Carboxy, Acetamido bedeuten, mit der Einschränkung, daß wenn n=0 ist, X₁, X₂ und X₃ nicht alle unabhängig ausgewählt sind aus der Gruppe bestehend aus Wasserstoff, Hydroxy und Nitro; oder
R₃S(O)ₘ
worin m=0, 1 oder 2 ist und R₃ Alkyl, Aryl oder Aralkyl bedeutet, oder gegebenenfalls mit Alkyl, Aryl oder Aralkyl substituiertes Carbamoyl bedeutet, oder R₁ und R₂ Methyl bedeuten und R 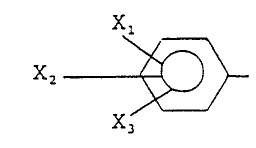 bedeutet, worin X₁, X₂ und X₃ wie oben definiert sind, mit der Einschränkung, daß einer der Reste X₂ und X₃ gegebenenfalls mit Alkyl, Aryl oder Aralkyl substituiertes Carbamoyl ist, wobei "Alkyl", "Alkoxy" und "Aralkyl" wie oben definiert sind; oder ein pharmazeutisch verträgliches Salz oder ein pharmazeutisch verträglicher Esters davon, ausgenommen
4-(Phenylmethylen)-3,5-heptandion,
4-(3,4-Dichlorphenylmethylen)-3,5-heptandion und
3-(Phenylmethylen)-2,4-hexandion.

29. Verbindung gemäß der allgemeinen Formel III 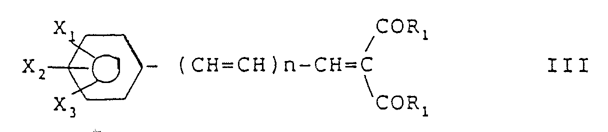 worin R₁ Methyl bedeutet, n 0 oder 1 ist, X₁ Wasserstoff, Hydroxy oder gegebenenfalls mit Aryl substituiertes Alkoxy bedeutet, und X₂
R₃S(O)ₘ
bedeutet, worin m 1 oder 2 ist und R₃ Alkyl, Aryl oder Aralkyl ist, und X₃ Wasserstoff, Nitro, Cyano, Halogen, Trifluormethyl, Formyl, Carboxy oder eine wie für X₂ definierte Gruppe bedeutet; oder X₂ und X₃ beide Cyano sind; oder X₂ Nitro und X₃ Formyl ist; oder worin wenn X₁ Hydroxy ist, X₂ Carboxy sein kann oder X₂ und X₃ beide Wasserstoff sein können, wenn n=0 und X₁ mit Aryl substituiertes Alkoxy ist oder wenn n=1 und X₁ Hydroxy oder gegebenenfalls mit Aryl substituiertes Alkoxy ist; wobei "Alkyl" eine Alkylgruppe mit vorzugsweise 1 bis 2 Kohlenstoffatomen bedeutet, "Alkoxy" einen wie oben definierten Alkylrest bedeutet, der an ein Sauerstoffatom gebunden ist, und "Aralkyl" eine wie oben definierte Alkylgruppe mit Aryl, vorzugsweise Phenyl, als Substituent bedeutet; oder ein pharmazeutisch verträgliches Salz oder ein pharmazeutisch verträglicher Ester davon.

30. 3-[(4-Methylsulfonylphenyl)methylen]-2,4-pentandion.

## Revendications (Revendications pour l'(les) Etat(s) contractant(s) suivant(s): AT, BE, CH, LI, DE, DK, FR, GB, IT, LU, NL, SE)

1. Utilisation d'un composé de formule (I) : 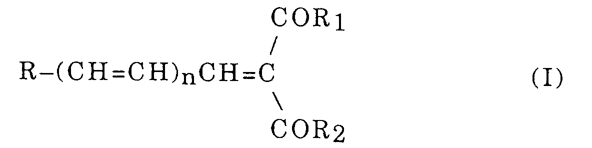 dans laquelle n = 0 ou 1, R₁ et R₂ sont indépendamment un groupe méthyle, éthyle ou cyclopropyle et R est un groupe phényle ou hétéroaryle éventuellement substitué : 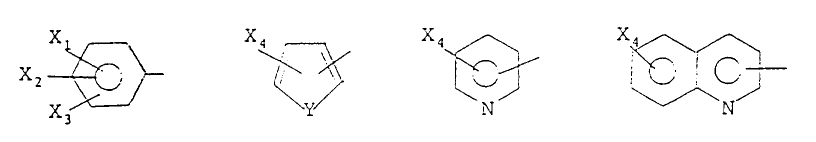 où X₁ est un atome d'hydrogène, un groupe hydroxy ou alcoxy qui est éventuellement substitué par un groupe aryle ; X₂ et X₃ sont indépendamment un atome d'hydrogène, un groupe nitro, cyano, halo, trifluorométhyle, formyle, carboxy, acétamido, ou
R₃S(O)ₘ
où m = 0, 1 ou 2, et R₃ est un groupe alkyle, aryle ou aralkyle ; ou carbamoyle qui est éventuellement substitué par un groupe alkyle, aryle ou aralkyle ; où le groupe "alkyle" est un groupe alkyle ayant de préférence 1 ou 2 atomes de carbone, le groupe "alcoxy" est un résidu alkyle tel que défini plus haute lié à un atome d'oxygène, et le groupe "aralkyle" est un groupe alkyle tel que défini plus haut, portant un groupe aryle, de préférence un groupe phényle comme substituant ; Y est un atome d'oxygène ou de soufre et X₄ est un atome d'hydrogène, un groupe nitro ou halo ; ou d'un sel ou ester de celui-ci acceptable du point de vue pharmaceutique dans la fabrication d'un médicament utile dans le traitement d'une affection intestinale inflammatoire.

2. Utilisation suivant la revendication 1, dans laquelle, dans le composé de formule (I), X₁ est un groupe hydroxy ou alcoxy.

3. Utilisation suivant les revendications 1 ou 2, dans laquelle, dans le composé de formule (I), l'un des X₂ et X₃ est un groupe nitro, cyano, trifluorométhyle, carboxy ou carbamoyle qui est substitué par un groupe aryle.

4. Utilisation suivant les revendications 1 à 3, dans laquelle le composé est :
la 3-(4-hydroxyphényl)-méthylène-2,4-pentanedione
la 3-(4-méthoxyphényl)-méthylène-2,4-pentanedione
la 3-(3-nitrophényl)-méthylène-2,4-pentanedione
la 3-(4-nitrophényl)-méthylène-2,4-pentanedione
la 3-(2-nitrophényl)-méthylène-2,4-pentanedione
la 3-[(4-(N-phénéthyl)-carboxamidophényl)-méthylène]-2,4-pentanedione
la 3-[(5-nitrofuryl)-méthylène]-2,4-pentanedione ou
la 2-[(4-carboxyphényl)-méthylène]-1,3-dicyclopropyl-1,3-propanedione.
ou un sel ou un ester de celles-ci acceptable du point de vue pharmaceutique.

5. Utilisation suivant les revendications 1 ou 3, dans laquelle le composé est la 3-(4-trifluorométhylphényl)-méthylène-2,4-pentanedione.

6. Utilisation suivant les revendications 1 ou 3, dans laquelle le composé est la 3-(4-cyanophényl)-méthylène-2,4-pentanedione.

7. Utilisation suivant les revendications 1 ou 3, dans laquelle le composé est la 3-(3-cyanophényl)-méthylène-2,4-pentanedione.

8. Utilisation suivant les revendications 1 ou 3, dans laquelle le composé est la 4-[(4-cyanophényl)-méthylène]-3,5-heptanedione.

9. Utilisation suivant les revendications 1 ou 3, dans laquelle le composé est la 2-[(4-cyanophényl)-méthylène]-1,3-dicyclopropyl-1,3-propanedione.

10. Utilisation suivant les revendications 1 ou 3, dans laquelle le composé est la 2-[(3-trifluorométhylphényl)-méthylène]-1,3-dicyclopropyl-1,3-propanedione.

11. Composé suivant la formule générale (II): 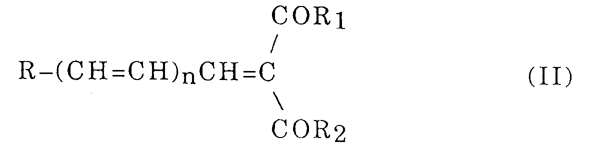 dans laquelle n = 0 ou 1, R₁ est un groupe méthyle, éthyle ou cyclopropyle et R₂ est un groupe éthyle ou cyclopropyle, et R est : 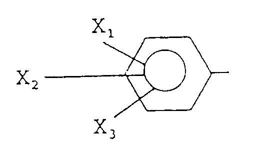 où X₁ est un atome d'hydrogène, un groupe hydroxy ou alcoxy qui est éventuellement substitué par un groupe aryle ; X₂ et X₃ sont indépendamment un atome d'hydrogène, un groupe nitro, cyano, halo, trifluorométhyle, formyle, carboxy, acétamido, à condition que lorsque n = 0, X₁, X₂ et X₃ ne soient pas tous indépendamment choisis dans le groupe consistant en atome d'hydrogène, groupe hydroxy et nitro ; ou:
R₃S(O)ₘ
où m = 0, 1 ou 2, et R₃ est un groupe alkyle, aryle ou aralkyle ; ou carbamoyle qui est éventuellement substitué par un groupe alkyle, aryle ou aralkyle ; ou bien R₁ et R₂ sont un groupe méthyle et R est : 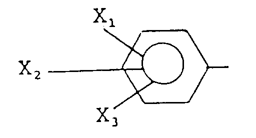 où X₁, X₂ et X₃ sont tels que définis plus haut, à condition que l'un des X₂ et X₃ soit un groupe carbamoyle qui est éventuellement substitué par un groupe alkyle, aryle ou aralkyle, où les termes "alkyle", "alcoxy" et "aralkyle" sont tels que définis ici ; ou sel ou ester de celui-ci acceptable du point de vue pharmaceutique, mais à l'exclusion de la 4-(phénylméthylène)-3,5-heptadione, de la 4-(3,4-dichlorophényl-méthylène)-3,5-heptanedione et de la 3-(phénylméthylène)-2,4-hexanedione.

12. Composé suivant la revendication 11, dans lequel dans le composé de formule (II), l'un des X₂ et X₃ est un groupe cyano ou trifluorométhyle.

13. 2-[(4-Acétamidophényl)-méthylène]-1,3-dicyclopropyl-1,3-propanedione.

14. 2-[(4-Méthoxyphényl)-méthylène]-1,3-dicyclopropyl-1,3-propanedione.

15. 3-[(4-Cyanophényl)-méthylène]-4-cyclopropyl-2,4-butanedione.

16. 2-[(4-Cyanophényl)-méthylène]-1,3-dicyclopropyl-1,3-propanedione.

17. 2-[(3-Trifluorométhylphényl)-méthylène]-1,3-dicyclopropyl-1,3-propanedione.

18. 2-[(4-Carboxyphényl)-méthylène]-1,3-dicyclopropyl-1,3-propanedione ou sel ou ester de celle-ci acceptable du point de vue pharmaceutique.

19. 4-[(4-Nitrophényl)-méthylène]-3,5 heptanedione.

20. 4-[(3-Cyanophényl)-méthylène]-3,5-heptanedione.

21. 4-[(4-Cyanophényl)-méthylène]-3,5-heptanedione.

22. 3-[(4-Carboxamidophényl)-méthylène]-2,4-pentanedione.

23. 3-[(4-(N-phénéthyl)-carboxamidophényl)-méthylène]-2,4-pentanedione.

24. Composé suivant la formule générale (III) : 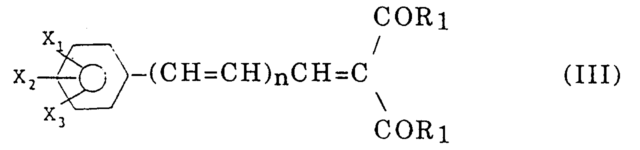 dans laquelle n = 0 ou 1, R₁ est un groupe méthyle et X₁ est un atome d'hydrogène, un groupe hydroxy ou alcoxy qui est éventuellement substitué par un groupe aryle, et X₂ est
R₃S(O)ₘ
où m = 1 ou 2, et R₃ est un groupe alkyle, aryle ou aralkyle et X₃ est un atome d'hydrogène, un groupe nitro, cyano, halo, trifluorométhyle, formyle, carboxy ou un groupe tel que défini pour X₂ ; ou X₂ et X₃ sont tous les deux un groupe cyano ; ou X₂ est un groupe nitro et X₃ est un groupe formyle , ou bien, lorsque X₁ est un groupe hydroxy, X₂ peut être un groupe carboxy, ou bien X₂ et X₃ peuvent tous les deux être un atome d'hydrogène lorsque n = 0 et X₁ est un groupe alcoxy substitué par un groupe aryle, ou lorsque n = 1, et X₁ est un groupe hydroxy ou alcoxy éventuellement substitué par un groupe aryle ; où le groupe "alkyle" est un groupe alkyle ayant de préférence 1 ou 2 atomes de carbone, le groupe "alcoxy" est un résidu alkyle tel que défini plus haut lié à un atome d'oxygène, et le groupe "aralkyle" est un groupe alkyle tel que défini plus haut, portant un groupe aryle, de préférence un groupe phényle comme substituant ; ou sel ou ester de celui-ci acceptable du point de vue pharmaceutique.

25. 3-[(4-Phénylsulfonylphényl)-méthylène]-2,4-pentanedione.

26. 3-[(4-Méthylsulfonylphényl)-méthylène]-2,4-pentanedione.

27. 3-[(4-Formyl-3-nitrophényl)-méthylène]-2,4-pentanedione.

## Revendications (Revendications pour l'(les) Etat(s) contractant(s) suivant(s): ES, GR)

1. Utilisation d'un composé de formule (I) : 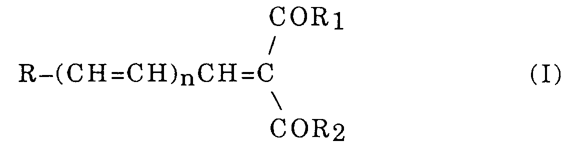 dans laquelle n = 0 ou 1, R₁ et R₂ sont indépendamment un groupe méthyle, éthyle ou cyclopropyle et R est un groupe phényle ou hétéroaryle éventuellement substitué : 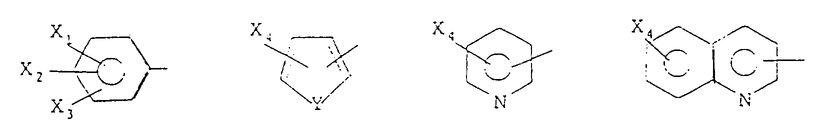 où X₁ est un atome d'hydrogène, un groupe hydroxy ou alcoxy qui est éventuellement substitué par un groupe aryle ; X₂ et X₃ sont indépendamment un atome d'hydrogène, un groupe nitro, cyano, halo, trifluorométhyle, formyle, carboxy, acétamido, ou
R₃S(O)ₘ
où m = 0, 1 ou 2, et R₃ est un groupe alkyle, aryle ou aralkyle ; ou carbamoyle qui est éventuellement substitué par un groupe alkyle, aryle ou aralkyle ; où le groupe "alkyle" est un groupe alkyle ayant de préférence 1 ou 2 atomes de carbone, le groupe "alcoxy" est un résidu alkyle tel que défini plus haut lié à un atome d'oxygène, et le groupe "aralkyle" est un groupe alkyle tel que défini plus haut, portant un groupe aryle, de préférence un groupe phényle comme substituant ; Y est un atome d'oxygène ou de soufre et X₄ est un atome d'hydrogène, un groupe nitro ou halo ; ou d'un sel ou ester de celui-ci acceptable du point de vue pharmaceutique dans la fabrication d'un médicament utile dans le traitement d'une affection intestinale inflammatoire.

2. Utilisation suivant la revendication 1, dans laquelle, dans le composé de formule (I), X₁ est un groupe hydroxy ou alcoxy.

3. Utilisation suivant les revendications 1 ou 2, dans laquelle, dans le composé de formule (I), l'un des X₂ et X₃ est un groupe nitro, cyano, trifluorométhyle, carboxy ou carbamoyle qui est substitué par un groupe aryle.

4. Utilisation suivant les revendications 1 à 3, dans laquelle le composé est :
la 3-(4-hydroxyphényl)-méthylène-2,4-pentanedione
la 3-(4-méthoxyphényl)-méthylene-2,4-pentanedione
la 3-(3-nitrophényl)-méthylène-2,4-pentanedione
la 3-(4-nitrophényl)-méthylène-2,4-pentanedione
la 3-(2-nitrophényl)-méthylène-2,4-pentanedione
la 3-[(4-(N-phénéthyl)-carboxamidophényl)-méthylène]-2,4-pentanedione
la 3-[(5-nitrofuryl)-méthylène]-2,4-pentanedione ou
la 2-[(4-carboxyphényl)-méthylène]-1,3-dicyclopropyl-1,3-propanedione.
ou un sel ou un ester de celles-ci acceptable du point de vue pharmaceutique.

5. Utilisation suivant les revendications 1 ou 3, dans laquelle le composé est la 3-(4-trifluorométhylphényl)-méthylène-2,4-pentanedione.

6. Utilisation suivant les revendications 1 ou 3, dans laquelle le composé est la 3-(4-cyanophényl)-méthylène-2,4-pentanedione.

7. Utilisation suivant les revendications 1 ou 3, dans laquelle le composé est la 3-(3-cyanophényl)-méthylène-2,4-pentanedione.

8. Utilisation suivant les revendications 1 ou 3, dans laquelle le composé est la 4-[(4-cyanophényl)-méthylène]-3,5-heptanedione.

9. Utilisation suivant les revendications 1 ou 3, dans laquelle le composé est la 2-[(4-cyanophényl)-méthylène]-1,3-dicyclopropyl-1,3-propanedione.

10. Utilisation suivant les revendications 1 ou 3, dans laquelle le composé est la 2-[(3-trifluorométhylphényl)-méthylène]-1,3-dicyclopropyl-1,3-propanedione.

11. Procédé pour la préparation d'un composé suivant la formule générale (II) : 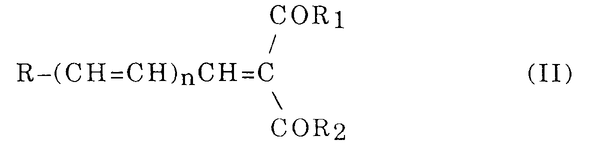 dans laquelle n = 0 ou 1, R₁ est un groupe méthyle, éthyle ou cyclopropyle et R₂ est un groupe éthyle ou cyclopropyle et R est : 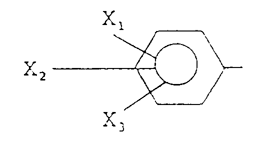 où X₁ est un atome d'hydrogène, un groupe hydroxy ou alcoxy qui est éventuellement substitué par un groupe aryle ; X₂ et X₃ sont indépendamment un atome d'hydrogène, un groupe nitro, cyano, halo, trifluorométhyle, formyle, carboxy, acétamido, à condition que lorsque n = 0, X₁, X₂ et X₃ ne soient pas tous indépendamment choisis dans le groupe consistant en atome d'hydrogène, groupe hydroxy et nitro ; ou :
R₃S(O)ₘ
où m = 0, 1 ou 2, et R₃ est un groupe alkyle, aryle ou aralkyle ; ou carbamoyle qui est éventuellement substitué par un groupe alkyle, aryle ou aralkyle ; ou bien R₁ et R₂ sont un groupe méthyle et R est : 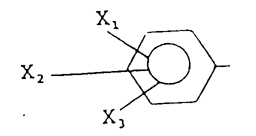 où X₁, X₂ et X₃ sont tels que définis plus haut, à condition que l'un des X₂ et X₃ soit un groupe carbamoyle qui est éventuellement substitué par un groupe alkyle, aryle ou aralkyle, où les termes "alkyle" "alcoxy" et "aralkyle" sont tels que définis ici ; ou d'un sel ou ester de celui-ci acceptable du point de vue pharmaceutique, mais à l'exclusion de la 4-(phénylméthylène)-3,5-heptadione, de la 4-(3,4-dichlorophényl-méthylène)-3,5-heptanedione et de la 3-(phénylméthylène)-2,4-hexanedione ledit procédé comprenant :
la réaction d'un composé de formule (IV) :
R₁-CO-CH₂-CO-R₂ (IV)
dans laquelle R₁ et R₂ sont tels que définis plus haut, avec un composé de formule (V) :
R-(CH=CH)ₙ-Z (V)
dans laquelle n et R sont tels que définis plus haut et Z est un groupe CHO ou -CH₂-Q, où Q est un atome d'halogène ou un autre groupe activé, en présence d'un catalyseur acide ou basique pour produire un composé de formule (II) ou un composé de formule (VI) : 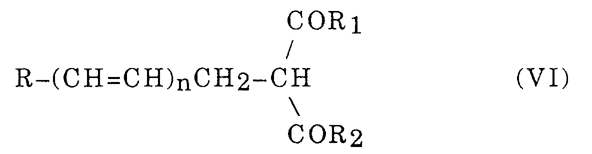 dans laquelle n, R₁, R₂ et R sont tels que définis plus haut,
l'halogénation de ce composé pour donner un composé de formule (VII) : 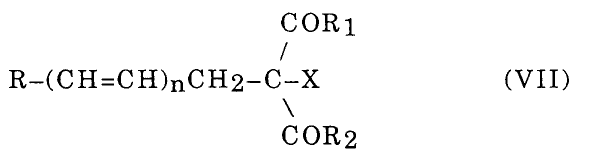 dans laquelle n, R₁, R₂ et R sont tels que définis plus haut et X est un atome d'halogène, et
la déshydrohalogénation du composé (VII) pour produire un composé de formule (II).

12. Procédé suivant la revendication 11, dans lequel, dans le composé de formule (II), l'un des X₂ et X₃ est un groupe cyano ou trifluorométhyle.

13. Procédé suivant la revendication 11 pour la préparation de la 2-[(4-acétamidophényl)-méthylène]-1,3-dicyclopropyl-1,3-propanedione.

14. Procédé suivant la revendication 11 pour la préparation de la 2-[(4-méthoxyphényl)-méthylène]-1,3-dicyclopropyl-1,3-propanedione.

15. Procédé suivant la revendication 11 pour la préparation de la 3-[(4-cyanophényl)-méthylène]-4-cyclopropyl-2,4-butanedione.

16. Procédé suivant la revendication 11 pour la préparation de la 2-[(4-cyanophényl)-méthylène]-1,3-dicyclopropyl-1,3-propanedione.

17. Procédé suivant la revendication 11 pour la préparation de la 2-[(3-trifluorométhylphényl)-méthylène]-1,3-dicyclopropyl-1,3-propanedione.

18. Procédé suivant la revendication 11 pour la préparation de la 2-[(4-carboxyphényl)-méthylène]-1,3-dicyclopropyl-1,3-propanedione ou d'un sel ou ester de celle-ci acceptable du point de vue pharmaceutique.

19. Procédé suivant la revendication 11 pour la préparation de la 4-[(4-nitrophényl)-méthylène]-3,5-heptanedione.

20. Procédé suivant la revendication 11 pour la préparation de la 4-[(3-cyanophényl)-méthylène]-3,5-heptanedione.

21. Procédé suivant la revendication 11 pour la préparation de la 4-[(4-cyanophényl)-méthylène]-3,5-heptanedione.

22. Procédé suivant la revendication 11 pour la préparation de la 3-[(4-carboxamidophényl)-méthylène]-2,4-pentanedione.

23. Procédé suivant la revendication 11 pour la préparation de la 3-[(4-(N-phénéthyl)-carboxamidophényl)-méthylène]-2,4-pentanedione.

24. Procédé pour la préparation d'un composé suivant la formule générale (III) : 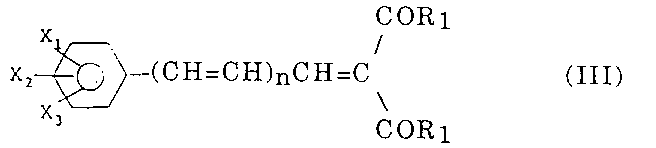 dans laquelle n = 0 ou 1, R₁ est un groupe méthyle et X₁ est un atome d'hydrogène, un groupe hydroxy ou alcoxy qui est éventuellement substitué par un groupe aryle, et X₂ est
R₃S(O)ₘ
où m = 1 ou 2, et R₃ est un groupe alkyle, aryle ou aralkyle et X₃ est un atome d'hydrogène, un groupe nitro, cyano, halo, trifluorométhyle, formyle, carboxy ou un groupe tel que défini pour X₂ ; ou X₂ et X₃ sont tous les deux un groupe cyano ; ou X₂ est un groupe nitro et X₃ est un groupe formyle ; ou bien, lorsque X₁ est un groupe hydroxy, X₂ peut être un groupe carboxy, ou bien X₂ et X₃ peuvent tous les deux être un atome d'hydrogène lorsque n = 0 et X₁ est un groupe alcoxy substitué par un groupe aryle, ou lorsque n = 1, et X₁ est un groupe hydroxy ou alcoxy éventuellement substitué par un groupe aryle ; où le groupe "alkyle" est un groupe alkyle ayant de préférence 1 ou 2 atomes de carbone, le groupe "alcoxy" est un résidu alkyle tel que défini plus haut lié à un atome d'oxygène, et le groupe "aralkyle" est un groupe alkyle tel que défini plus haut, portant un groupe aryle, de préférence un groupe phényle comme substituant ; ou d'un sel ou ester de celui-ci acceptable du point de vue pharmaceutique, ledit procédé comprenant :
la réaction d'un composé de formule (IV) :
R₁-CO-CH₂-CO-R₂ (IV)
dans laquelle R₁ et R₂ sont tels que définis plus haut, avec un composé de formule (V) :
R-(CH=CH)ₙ-Z (V)
dans laquelle n et R sont tels que définis plus haut et Z est un groupe CHO ou -CH₂-Q, où Q est un atome d'halogène ou un autre groupe activé, en présence d'un catalyseur acide ou basique pour produire un composé de formule (III) ou un composé de formule (VI) : 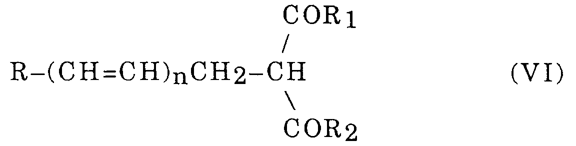 dans laquelle n, R₁ et R₂ sont tels que définis plus haut et R est un groupe phényle substitué par X₁, X₂ et X₃ tels que définis plus haut,
l'halogénation de ce composé pour donner un composé de formule (VII) : 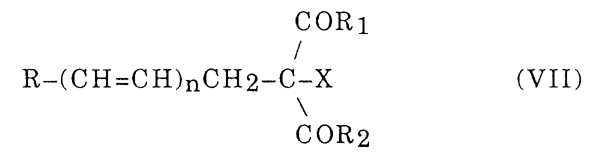 dans laquelle n, R₁, R₂ et R sont tels que définis plus haut et X est un atome d'halogène, et
la déshydrohalogénation du composé (VII) pour produire un composé de formule (I).

25. Procédé suivant la revendication 24 pour la préparation de la 3-[(4-phénylsulfonylphényl)-méthylène]-2,4-pentanedione.

26. Procédé suivant la revendication 24 pour la préparation de la 3-[(4-méthylsulfonylphényl)-méthylène]-2,4-pentanedione.

27. Procédé suivant la revendication 24 pour la préparation de la 3-[(4-formyl-3-nitrophényl)-méthylène]-2,4-pentanedione.

28. Composé suivant la formule générale (II) : 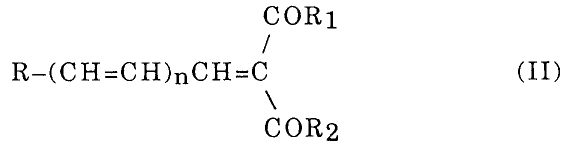 dans laquelle n = 0 ou 1, R₁ est un groupe méthyle, éthyle ou cyclopropyle et R₂ est un groupe éthyle ou cyclopropyle, et R est : 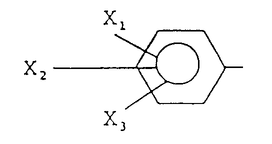 où X₁ est un atome d'hydrogène, un groupe hydroxy ou alcoxy qui est éventuellement substitué par un groupe aryle ; X₂ et X₃ sont indépendamment un atome d'hydrogène, un groupe nitro, cyano halo, trifluorométhyle, formyle, carboxy, acétamido, à condition que lorsque n = 0, X₁, X₂ et X₃ ne soient pas tous indépendamment choisis dans le groupe consistant en atome d'hydrogène, groupe hydroxy et nitro ; ou :
R₃S(O)ₘ
où m = 0, 1 ou 2, et R₃ est un groupe alkyle, aryle ou aralkyle ; ou carbamoyle qui est éventuellement substitué par un groupe alkyle, aryle ou aralkyle ; ou bien R₁ et R₂ sont un groupe méthyle et R est : 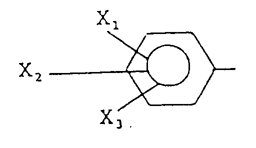 où X₁, X₂ et X₃ sont tels que définis plus haut, à condition que l'un des X₂ et X₃ soit un groupe carbamoyle qui est éventuellement substitué par un groupe alkyle, aryle ou aralkyle, où les termes "alkyle", "alcoxy" et "aralkyle" sont tels que définis ici ; ou sel ou ester de celui-ci acceptable du point de vue pharmaceutique, mais à l'exclusion de la 4-(phénylméthylène)-3,5-heptadione, de la 4-(3,4-dichlorophényl-méthylène)-3,5-heptanedione et de la 3-(phénylméthylène)-2,4-hexanedione.

29. Composé suivant la formule générale (III) : 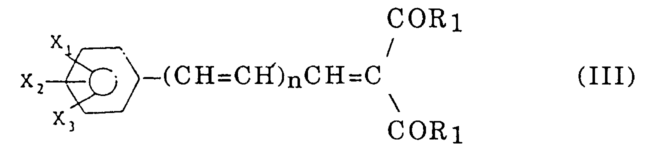 dans laquelle n = 0 ou 1, R₁ est un groupe méthyle et X₁ est un atome d'hydrogène, un groupe hydroxy ou alcoxy qui est éventuellement substitué par un groupe aryle, et X₂ est
R₃S(O)ₘ
où m = 1 ou 2, et R₃ est un groupe alkyle, aryle ou aralkyle et X₃ est atome d'hydrogène, un groupe nitro, cyano, halo, trifluorométhyle, formyle, carboxy ou un groupe tel que défini pour X₂ ; ou X₂ et X₃ sont tous les deux un groupe cyano ; ou X₂ est un groupe nitro et X₃ est un groupe formyle , ou bien, lorsque X₁ est un groupe hydroxy, X₂ peut être un groupe carboxy, ou bien X₂ et X₃ peuvent tous les deux être un atome d'hydrogène lorsque n = 0 et X₁ est un groupe alcoxy substitué par un groupe aryle, ou lorsque n = 1, et X₁ est un groupe hydroxy ou alcoxy éventuellement substitué par un groupe aryle ; où le groupe "alkyle" est un groupe alkyle ayant de préférence 1 ou 2 atomes de carbone, le groupe "alcoxy" est un résidu alkyle tel que défini plus haut lié à un atome d'oxygène, et le groupe "aralkyle" est un groupe alkyle tel que défini plus haut, portant un groupe aryle, de préférence un groupe phényle comme substituant ; ou sel ou ester de celui-ci acceptable du point de vue pharmaceutique.

30. 3-[(4-Méthylsulfonylphényl)-méthylène]-2,4-pentanedione.
